## Europäisches Patentamt

(19) **European Patent Office**

**Office européen des brevets**

(11) Veröffentlichungsnummer: **0 074 428**

**B1**

(12) **EUROPÄISCHE PATENTSCHRIFT**

(45) Veröffentlichungstag der Patentschrift:
08.04.87

(51) Int. Cl.⁴: **G 01 N 21/47**, A 61 B 5/14

(21) Anmeldenummer: **81107264.4**

(22) Anmeldetag: **15.09.81**

(54) **Verfahren und Vorrichtung zur quantitativen Bestimmung gelöster Substanzen in Ein- und Mehrkomponentensystemen durch Laser-Lichtstreuung.**

(43) Veröffentlichungstag der Anmeldung:
23.03.83 Patentblatt 83/12

(45) Bekanntmachung des Hinweises auf die Patenterteilung:
08.04.87 Patentblatt 87/15

(84) Benannte Vertragsstaaten:
AT CH DE FR GB LI NL

(56) Entgegenhaltungen:
EP-A-0 030 610
DE-A-2 709 866
US-A-3 310 680
US-A-3 659 946
US-A-3 786 261
US-A-4 118 625
US-A-4 146 799

BIOMEDIZINISCHE TECHNIK, Band 25, Nr. 1,2, Januar-Februar 1980, Seiten 26-32, Berlin, DE, H. BRAUNER et al.:"Glukosebestimmung - Stand und Perspektiven"
APPLIED OPTICS, Band 18, Nr. 3, 1. Februar 1979, Seiten 303-311, New York, USA, S.J.MORRIS et al.:"Dynamics of structural chnges inbiological particles from rapid light scattering measurements"
ARCHIEF FÜR TECHNISCHE MESSUNG, Band 462, Lieferung 464, September 1974, Seiten V-462, 1-3, Darmstadt, DE., E. DELAGO: "Messung kleiner Trübungen"

(73) Patentinhaber: **Müller, Arno, Dr. Dipl.- Phys.,**
**Gartenhalde 22, D-7900 Ulm- Mähringen (DE)**

(72) Erfinder: **Müller, Arno, Dr. Dipl.- Phys.,**
**Gartenhalde 22, D-7900 Ulm- Mähringen (DE)**

(74) Vertreter: **Patentanwälte Beetz sen. - Beetz jun.**
**Timpe - Siegfried - Schmitt- Fumian,**
**Steinsdorfstrasse 10, D-8000 München 22 (DE)**

# 0 074 428

## Beschreibung

Die Erfindung betrifft ein Verfahren sowie eine hochempfindliche Vorrichtung zur quantitativen Bestimmung gelöster niedermolekularer und hochmolekularer Substanzen, besonders von Glucose, in mehrkomponentigen wäßrigen und nichtwäßrigen Lösungen und insbesondere in biologischen Systemen in vitro und in vivo an bzw. in lebenden Organismen durch Lichtstreuung.

Die Erfindung beruht auf dem physikalischen Prinzip der Lichtstreuung. Es ist bekannt, daß durch Wechselwirkung z.B. eines monochromatischen Lichtstrahls mit einem Molekül eine von Art und Struktur des Moleküls abhängige Lichtstreuung auftritt. In einem Mehrkomponentensystem z.B. einem biologischen System wie etwa dem menschlichen Blut, sind jedoch sehr viele verschiedene Moleküle unterschiedlicher Größe enthalten, so daß von vornherein eine selektive Bestimmung nur einer Komponente, z.B. der Glucose, durch Lichtstreuung nicht möglich erscheint.

Aus der US-A-3 310 680 ist bereits ein Streulicht-Photometer bekannt, das zur Bestimmung der Konzentration kolloidaler Lösungen vorgesehen ist, wobei die Streulichtintensität in einem vorgegebenen Winkelbereich sowie die Zentralstrahlintensität gemessen werden, und mit einem Quotientenbildner das Verhältnis der entsprechenden beiden Meßsignale gebildet wird, das der Konzentration des Kolloids proportional ist. Diese Vorrichtung eignet sich nicht zur Konzentrationsbestimmung bestimmter Komponenten in Systemen mit mehreren zu bestimmenden Komponenten, insbesondere der der vorliegenden Erfindung zugrundeliegenden Art. Entsprechend ist auch eine Verwendbarkeit zur Konzentrationsbestimmung niedermolekularer oder hochmolekularer gelöster Substanzen in Mehrkomponentensystemen nicht angesprochen.

Der Erfindung liegt die Aufgabe zugrunde, ein Verfahren sowie eine hochempfindliche Vorrichtung zur quantitativen Bestimmung gelöster niedermolekularer sowie hochmolekularer Substanzen, wie etwa von Proteinen, sowie von Blutkörperchen und Blutzellen, insbesondere von Glucose in mehrkomponentigen wäßrigen und nichtwäßrigen Lösungen und besonders in biologischen Systemen in vitro und in vivo an bzw. in lebenden Organismen anzugeben, wobei eine selektive und quantitative Bestimmung einer oder mehrerer bestimmter nieder- oder Hochmolekularer Substanzen oder von Blutkörperchen bzw. Blutzellen auch in Gegenwart anderer gelöster bzw. disperser Substanzen möglich sein soll.

Die Aufgabe wird nach den kennzeichnenden Merkmalen der Ansprüche 1 und 15 gelöst.

Das erfindungsgemäße Verfahren erlaubt die quantitative Bestimmung der Konzentration niedermolekularer und hochmolekularer Substanzen sowie von Blutkörperchen und Blutzellen in Mehrkomponentensystemen, wobei sich die Einzelkomponenten in den Lineardimensionen der Moleküle, ihrer Struktur und/oder Zusammensetzung und/oder in der Molekülmasse hinreichend unterscheiden, durch

(a) Lichtstreuung an der Probe und Analyse der Streukurvenform unter Ermittlung mindestens eines Streuwinkelbereichs pro zu bestimmende Komponente, in dem die stärkste Abhängigkeit der Intensität der Streustrahlung von der Konzentration der jeweiligen Komponente vorliegt,

(b) Korrektur der gemessenen Intensität der jeweiligen Streustrahlung mit der gemessenen Intensität des Zentralstrahls
und

(c) Zuordnung der korrigierten Meßgröße zur Konzentration der zu bestimmenden Komponente.

Die erfindungsgemäße Vorrichtung zur Durchführung dieses Verfahrens geht gattungsgemäß von dem Streulichtphotometer gemäß der US-A 33 10 680 aus und umfaßt

- eine Lichtquelle,
- ein Probenteil,
- einen oder mehrere Lichtdetektoren, welche die Intensität des Zentralstrahls und der Streustrahlung in mindestens einem vorgegebenen Winkelbereich erfassen,
- eine elektronische Signalverarbeitungseinrichtung zur Korrektur der Meßsignale,
- eine Stromversorgung
sowie
- eine Signalanzeigeeinrichtung oder einer Signalausgabevorrichtung,
und ist gekennzeichnet durch
- einen im Prinzip etwa U-förmigen Träger mit einem in seinem Bodenbereich vorgesehenen Gelenk mit einer Feder, die die beiden Seitenteile des Trägers unter einem Federdruck gegeneinander drückt, und einem derartigen Abstand der beiden Seitenteile voneinander, daß in den Zwischenraum zwischen den beiden Seitenteilen des Trägers ein menschliches Ohrläppchen eingeführt und darin festgehalten werden kann,
- eine an bzw. in einem Seitenteil des Trägers vorgesehene, gegebenenfalls in einem Kühlblock angeordnete Laserdiode als Lichtquelle,
- einen über der Laserdiode angebrachten Umlenkspiegel, der das von der Laserdiode emittierte Licht um etwa 90° in etwa waagerechte Richtung umlenkt,
und
- ein gegenüber dem Umlenkspiegel am anderen Seitenteil des Trägers vorgesehenes optisches Empfangsteil mit mindestens einem Lichtdetektor, das die Intensität des Zentralstrahls und der Streustrahlung des durch das in dem Träger eingeführte Ohrläppchen hindurchgehenden bzw. gestreuten Lichts der Laserdiode in mindestens einem vorgegebenen Winkelbereich erfaßt und entsprechende elektrische Signale liefert,
- wobei die elektronische Signalverarbeitungseinrichtung, die Signalanzeigeeinrichtung und/oder die

2

Signalausgabeeinrichtung sowie die Stromversorgung jeweils mit dem Träger integriert oder von ihm unabhängig vorgesehen sind,

Vorteilhafte Weiterbildungen der Erfindung sind in den abhängigen Anspruchen angegeben.

Aus der EP-Al 30 610 ist ferner ein Polarimeter, insbesondere zur Glucosebestimmung, bekannt, dessen optischer Teil ohrclipartig ausgebildet ist.

Das erfindungsgemäße Verfahren kann besonders günstig verletzungsfrei an lebenden Organismen an einem geeigneten Körperteil durchgeführt werden, beispielsweise durch transcutane Messung an Hautfalten und insbesondere am Ohrläppchen. Die erfindungsgemäße Vorrichtung ist entsprechend vorzugsweise in miniaturisierter Form ausgebildet und stellt besonders bevorzugt eine ohrclipartige Anordnung dar, mit der beispielsweise die Blutglucosekonzentration verletzungsfrei transcutan in vivo gemessen werden kann.

Die Erfindung geht von der überraschenden Feststellung aus, daß entgegen der fachmännischen Erwartung gelöste niedermolekulare sowie hochmolekulare Komponenten wie etwa Proteine und Hämoglobin sowie auch größere Partikel, wie Blutkörperchen bzw. Blutzellen, in Mehrkomponentensystemen und insbesondere in biologischen Systemen durch Laser-Lichtstreuung quantitativ und selektiv bestimmt werden können.

Das Prinzip des erfindungsgemäßen Verfahrens wird im folgenden unter Bezug auf die Bestimmung von Glucose beispielhaft erläutert.

Die Größe des Glucosemoleküls liegt zwischen der Große von Ionen wie $K^+$, $Na^+$, $Ca^{2+}$ udgl. und der Größe von Makromolekülen wie etwa Lipiden, Proteinen udgl.sowie den Blutzellen. Während für die obigen Ionen vor wiegend die Weitwinkelstreuung charakteristisch ist, ist für die Makromoleküle in erster Linie die Kleinwinkelstreuung kennzeichnend. Für in der Größe dazwischen liegende Moleküle wie die Glucose ist ein Streubereich maßgebend, der zwischen der Weitwinkelstreuung und der Kleinwinkelstreuung liegt.

Die Methodik der Lichtstreuungsmessung an kleinen Teilchen ist sowohl für Gase als auch für LLösungen theoretisch bestens untersucht (vgl. H.C. van de Hulst, Light Scattering by Small Particles, John Wiley and Sons, New York, 1957, Ch. Tanford, Physical Chemistry of Macromolecules, John Wiley and Sons, New York, 1961; W. Holzmüller, K. Altenburg, Physik der Kunststoffe, Akademie-Verlag, Berlin, 1961, und E.P. Geiduschek und Holtzer, Advances in Biol. and Med. Phys. 6 (1958) 431); aufgrund der vorhandenen theoretischen Grundlagen muß geprüft werden, ob die jeweiligen Voraussetzungen für den in Frage stehenden Winkelbereich gültig sind.

Das Verhältnis der Streulichtintensität I zur Intensität $I_0$ des eingestrahlten Lichts ist

$$\frac{I}{I_o} = \frac{2\pi^2 n_o^2 (dn/dc)^2 M c}{N \cdot \lambda^4 \cdot r^2} (1 + \cos^2 \theta) \quad (1);$$

in dieser Gleichung bedeuten:

$I_0$ = Intensität des eingestrahlten Lichts,

I = Intensität des Streulichts,

$n_0$ = Brechungsindex des Lösungsmittels,

c = molare Konzentration,

dn/dc = Änderung des Brechungsindex der Lösung mit der Konzentration,

M = Molekülmasse des gelösten Stoffs,

N = Avogadrosche Zahl = $\frac{\text{Loschmidtsche Zahl}}{\text{Molvolumen}}$,

r = Abstand vom Streuzentrum,

$\gamma$ = Wellenlänge des eingestrahlten Lichts,

$\theta$ = Winkel in Vorwärtsrichtung, gemessen zum Primärstrahl.

Üblicherweise wird in der Literatur der sog. Streuvektor

$$\vec{s}$$

eingeführt, der definiert ist durch

$$|\vec{s}| = \frac{2 \sin \theta}{\lambda}$$

Bei der Laser-Lichtstreuung ist für die Zentrallinie die exponentielle Näherung sinnvoll:

$$I^c(s) = (\zeta_0 V_0)^2 \cdot e^{-(4\pi^2 \cdot s^2 \cdot R^2_D)} \qquad (2),$$

wobei bedeuten:
$\zeta_0 V_0$ = Zahl der streuenden Teilchen,
$R_D$ = Streumassenradius
(vgl.W. Koechner, Solid-State Laser Engineering, Springer-Verlag New York, 1976; R. Beck, W. Englisch, K.Cürs, Table of Laser Lines in Gases and Vapors, Springer-Verlag Berlin, 1976, und H. Kressel, Editor, Topics in Applied Physics, Vol. 39, Semiconductor Devices for Optical Communication, Berlin, Heidelberg, New York, 1980).

Der Streumassenradius der Zentrallinie ist hier in seiner physikalischen Bedeutung ein Charakteristikum für die Laserlinie und das reine Lösungsmittel (z.B. Wasser). Aus dem Verhältnis der Intensitäten bei $s = 0$ und beispielsweise $s = 0,4$ läßt sich der Streumassenradius bestimmen. Für Glucose ergibt sich bei einer Wellenlänge von 0,829 µm ein Verhältnis von 47,5:1; hieraus folgt ein Streumassenradius $R_D$ von 0,233 µm.

Um die gesamte Streustrahlung zu erfassen, müssen die auftretenden Interferenzen durch einen zusätzlichen Term berücksichtigt werden. Für die Interferenzfunktion ergibt sich mit der gleichen Näherung, die bei Gleichung (2) verwendet wurde, folgende Beziehung:

$$I'(s) = (\zeta_1 V_1)^2 \cdot \left[ e^{-\frac{4\pi^2 \cdot s^2 \cdot R_1^2}{10}} - e^{-\frac{4\pi^2 \cdot s^2 \cdot R_2^2}{10}} \right] \qquad (3);$$

die Konstanten R besitzen dabei folgende Bedeutung:
$R_1$ = innerer Radius der Streuzentren,
$R_2$ = äußerer Radius der Streuzentren,
wobei die Streuzonen unterschiedliche Dichte aufweisen.

Die Gesamtintensität setzt sich mit guter Näherung additiv aus den Teilintensitäten zusammen. Da jedoch mit zunehmender Interferenz die Amplitude stets abnimmt, muß I'(s) ein negatives Vorzeichen besitzen. Damit ergibt sich für die gesamte Streuintensität der Lösung die Beziehung
$I(s) = I^o(s) - I'(s)$ (4).

Im Prinzip sind die Konstanten $R_1$ und $R_2$ von der Konzentration der jeweiligen Lösung abhängig. Da diese Abhängigkeit jedoch bei jeder Komponente einer Mehrkomponentenlösung wegen der unterschiedlichen Wechselwirkungen sehr verschieden sein kann, wobei auch Wechselwirkungen von Molekülen unterschiedlicher Art miteinander zu berücksichtigen sind, ist diese Abhängigkeit nur sehr schwer zu ermitteln.

Es ist deshalb sinnvoll, den Einfluß der Konzentration der gelösten Substanz, z.B. von Glucose, durch einen formalistischen Produktansatz der Form
$I(s) = I^o(s) - F(c) - I'(s)$ (5)
zu berücksichtigen, wobei für F(c) folgender Potenzansatz sinnvoll erscheint:
$F(c) = A.c^B$,
wobei A und B empirisch zu bestimmende Konstante darstellen.

Zunächst sollen zur Erläuterung zwei Fälle mit unterschiedlichem Streuverhalten unterschieden werden: 1. Lösungen mit mer einer gelösten Komponenete, die bestimmt werden soll, und 2. Lösungen mit mehreren Komponenten, von denen eine oder mehrere bestimmt werden sollen, z.B. biologische Systeme.

1. Lichstreuung an Lösungen mit nur einer gelösten, zu bestimmenden Komponente, z.B. Lösungen von Glucose in Wasser:

Aus Formel (1) resultiert Proportionalität der Intensität der Streustrahlung zur Konzentration der streuenden Substanz; diese Proportionalität ist zumindest für niedrige Konzentrationen experimentell zu erwarten, da bei hohen Konzentrationen die Wechselwirkungen der gelösten Moleküle untereinander zu Interferenzerscheinungen führen können (vgl. S. Flügge (Herausgeber) Handbuch der Physik, Band XXXII (Strukturforschung), Prof. Dr. W. W. Beeman, Size of Particles and Lattice Defects, Berlin, Heidelberg, Göttingen, 1957).

Für Hämoglobinlösungen in Wasser wurde erst im Konzentrationsbereich von 10 % und mehr eine Abnahme der Intensität der Streustrahlung bei einem Streuwinkel von etwa 1° mit steigender Konzentration experimentell festgestellt.

Für den Fachmann bestand daher bereits für derartige einfachen Systeme keinerlei Erwartungshaltung dafür, daß sich niedermolekulare Substanzen wie etwa Glucose in wäßrigen Lösungen auch bei niedrigen Konzentrationen durch Lichtstreuung quantitativ bestimmen lassen.

Es ist im Rahmen der Erfindung völlig überraschend, daß sich selbst bei Konzentraticnen von Glucose um 1 % in Wasser bei Verwendung eines He-Ne-Lasers (Leistung etwa 8 mW) als Lichtquelle im Winkelbereich von 0,5 bis 1,5° eine Abnahme der Intensität der Streustrahlung mit der Konzentration ergibt, wobei das Maximun der Intensitätsänderung bei etwa 1,5° liegt.

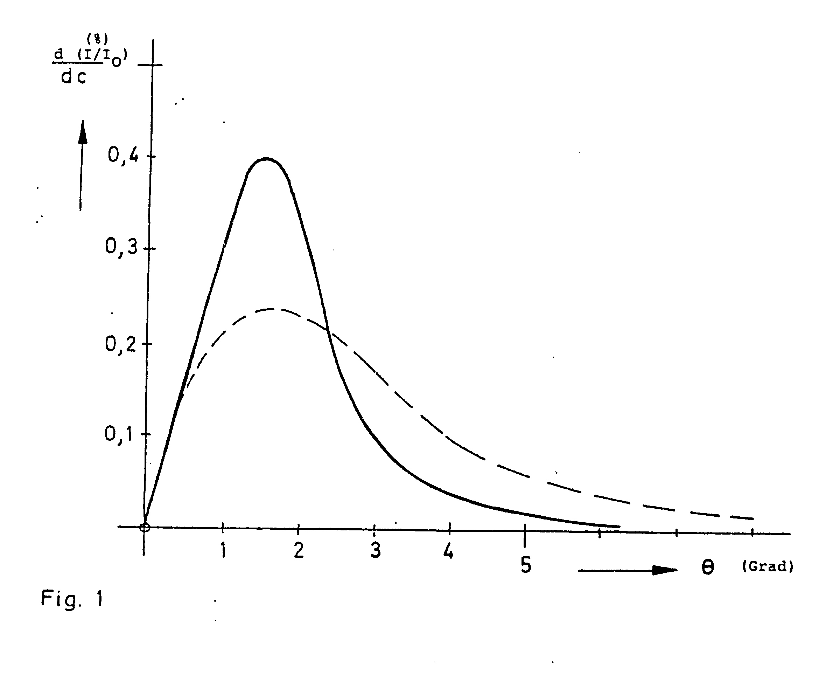

Derartige überraschende Meßergebnisse sind in Fig. 1 dargestellt, wo an der Ordinate die Änderung der Intensität mit der Glucosekonzentration und an der Abszisse der Streuwinkel aufgetragen sind.

Aus Gleichung (5) ergibt sich für die Änderung der Intensität mit der Glucosekonzentration die Beziehung

$$\frac{d\,I(s)}{dc} = -\frac{d\,F(c)}{dc} \cdot I'(s) = C \cdot I'(s) \quad (6)$$

mit $C = -A \cdot B \cdot c^{B-1}$.

Diese Funktion ist mit den Parametern $R_1 = 3,0\ \mu m$, $R_2 = 3,12\ \mu m$ und $C(\rho_1 V_1/\rho_0 V_0)^2 = 0,6$ in Fig. 1 für eine 1-%ige Lösung von Glucose in Wasser als gestrichelte Linie eingezeichnet. Die Meßergebnisse sind als durchgezogene Kurve eingetragen. Fig. 1 zeigt entsprechend, daß der theoretische Ansatz von Gleichung (6) eine zumindest gualitativ mit der Messung hinsichtlich der Winkellage des Maximums der Intensitätsänderung gute Übereinstimmung liefert.

Eine bessere Übereinstimmung eines theoretischen Ansatzes mit den in Fig. 1 dargestellten Meßwerten kann durch eine differenziertere Ausarbeitung der Funktion F(c) und/oder durch Untersuchung der Abhängigkeit der Konstanten $R_1$ und $R_2$ Von der Konzentration der Lösung erzielt werden.

Eine mögliche physikalische Interpretation der oben erläuterten überraschenden Lichtstreuerscheinungen in niedrigkonzentrierten Lösungen niedermolekularer Substanzen ist, daß selbst bei kleinen Konzentrationen assoziative Wechselwirkungen zwischen den Glucosemolekülen stattfinden, wobei diese sich zu Assoziaten zusammenlagern und auf diese Weise zu einer Streustrahlung führen, die sich nicht aus den Dimensionen eines Einzelmoleküls ableiten läßt.

Aus den gemessenen Intensitäten bei 1,5° ergibt sich durch Verhältnisbildung aus den Gleichungen (2) und (3) die Beziehung

$$\frac{I'(s_1)}{I^0(s_1)} = \frac{(\rho_1 V_1)^2 \cdot 0,0144}{(\rho_0 V_0)^2 \cdot 0,998} = 0,003;$$

hieraus folgt

$\frac{n}{n} = 0,5$.

Dies bedeutet, daß etwa die Hälfte aller Moleküle einen Beitrag zu dieser Streustrahlung leisten.

2. Lichtstreuung an Lösungen z.B. biologische Systeme wie Blut:

Bei solchen Mehrkomponentensystemen und insbesondere biologischen Systemen ist die Wechselwirkung der Moleküle untereinander mit Sicherheit nicht mehr vernachlässigbar. Hierdurch ergibt sich eine zwar nicht grundsätzlich unterschiedliche Form der Streukurve, jedoch ein um Größenordnungen unterschiedliches Verhältnis der Intensitäten bei verschiedenen Streuwinkeln. Während bei einfachen Lösungen etwa beim oben erläuterten Fall 1 ein Intensitätsverhältnis von 0,003 bei einem Streuwinkel von etwa 1,5° vorliegt, sind bei den oben definierten Mehrkomponentensystemen bei gleichem Streuwinkel Intensitäten zu erwarten, deren Verhältnis etwa gleich 1 ist. Theoretisch kann die Intensität des Zentralstrahls unter der Intensität der Streustrahlung bei bestimmten Winkeln liegen (vgl. A. Guinier, X-Ray Diffraction, W. H. Freeman and Company, San Francisco, 1963). In dieser Druckschrift sind die Streuintensitäten als Funktion der Größe $V_M/V_0$ untersucht und berechnet, wobei $V_M$ das Volumen des betrachteten Moleküls und $V_0$ sein freies Volumen, das umgekehrt proportional zur Konzentration ist, bedeuten. Für $V_M/V_0 = 0$ ergibt sich die übliche Form einer Streukurve (Glockenkurvenform), während für $V_M/V_0$) 0,25 die Intensität des Zentralstrahls mit wachsendem Streuwinkel zunächst zunimmt und dann erst abnimmt.

Untersucht man nun diese Größe z.B. für Verschiedene Blutkomponenten, so stellt man fest, daß sie bei Glucose den Wert von etwa $10^{-3}$ besitzt/ wobei zahlreiche andere Blutkomponenten ähnlich kleine Werte aufweisen, während die Erythrocyten mit 0,4, Proteine mit 0,1 und Hämoglobin mit 0,3 für diese Betrachtungen Werte in einem relevanten Bereich besitzen.

Für eine Konzentrationsbestimmung im Blut eignen sich daher die Erythrocyten, die Proteine sowie etwa das

5

Hämoglobin, woraus sich erfindungsgemäß eine neuartige transcutane Bestimmung der Erythrocytenzahl, der Hb-Werte udgl. wie auch eine quantitative Bestimmung von Glucose im Blut ergibt, die diese Weise zunächst nicht möglich erschien.

Aufgrund der Erfahrungen von Streulichtmessungen bei niedrig konzentrierten Glucoselösungen in Wasser wurde im Rahmen der Erfindung ein Versuch zur Bestimmung der Glucose in mehrkomponentigen Lösungen (wie z.B. Blut) unternommen, der wider Erwarten erfolgreich war.

Die Erfindung geht also von der völlig überraschenden Feststellung aus, daß auch niedermolekulare Substanzen, wie Glucose, in Mehrkomponentensystemen, wie Blut, durch Laser-Lichtstreuung quantitativ bestimmt werden können.

Dieser Befund läßt sich theoretisch wie folgt erklären:

Ändert sich die Konzentration einer niedermolekularen Komponente, wie etwa Glucose im Blut, so ändern sich auch die freien Volumina der übrigen Komponenten und damit die Verhältnisse $V_{Mi}/V_{oi}$ (i = 1, 2, 3 ...), wobei die mit i indizierten Komponenten Werte im theoretisch relevanten Bereich $0,1 \leq \frac{V_M}{V_o} \leq 0,5$) besitzen; somit ändert sich auch die Form der Streukurve.

Zur sicheren Identifizierung der zu analysierenden niedermolekularen Komponente, z.B. der Glucose, müssen allerdings noch zusätzliche Informationen herangezogen werden bzw. bestimmte Voraussetzungen erfüllt sein. Dies bedeutet vor allem, daß die Konzentrationen der relevanten Komponenten konstant bleiben müssen. Die Konstanz der Blutzellenkonzentration ist bekannt (Wissenschaftliche Tabellen Geigy, Teilband Hämatologie und Humangenetik, CIBA-GEIGY AG, Basel, 8. Auflage 1979). Eine Beeinflussung des freien Volumens der Blutzellen durch unterschiedliche Glucosekonzentrationen ist jedoch ohnehin wegen des um etwa 7 Größenordnungen verschiedenen Molekül- bzw. Partikelvolumens unwahrscheinlich. Die Proteinkonzentration schwankt andererseits beim Menschen zwischen einem Lebensalter von etwa 20 bis etwa 70 Jahren nur um etwa 2 %, wie ebenfalls aus Wissenschaftliche Tabellen Geigy, Teilband Hämarologie und Humangenetik, CIBA-GEIGY AG, Basel, 8. Auflage, 1979 hervorgeht. Diese Schwankung ist dementsprechend extrem gering. Allerdings sind pathologische Veränderungen bekannt, die erforderlichenfalls überwacht bzw berücksichtigt werden müssen; im Normalfall kann jedoch, beispielsweise auch bei Diabetikern, von einer Konstanz der Proteinkonzentration ausgegangen werden.

Die Hämoglobinkonzentration wird durch Regulationsmechanismen ebenfalls weitgehend konstantgehalten.

Alle übrigen Blutkomponenten können ferner entweder wegen ihrer geringen Konzentration oder wegen ihres geringen Eigenvolumens in Verbindung mit ihrer geringen Konzentration unberücksichtigt bleiben.

Die noch als potentielle Störsubstanzen in Frage kommenden kleinen Ionen Na+, K+ udgl. werden ebenfalls durch sehr empfindliche biologische Regelmechanismen auf konstanter Konzentration gehalten.

Erfindungsgemäß kann also die Bestimmung der Konzentration nur einer Komponente in mehrkomponentigen Lösungen, z.B. von Glucose im Blut, unter den oben genannten Voraussetzungen durch Laser-Lichtstreuung und Analyse der Form der Streukurve bzw. durch entsprechende Intensitätsmessungen erfolgen, wobei vermutlich für die Meßbarkeit die Veränderung des freien Volumens der relevanten Komponenten verantwortlich ist; diese theoretische Begründung ist allerdings derzeit noch nicht gesichert.

Die Messung der Intensität der Streustrahlung zur Analyse der Streukurvenform kann in Vorwärtsrichtung unter einem oder mehreren Streuwinkeln im gesamten Streuwinkelbereich von 0,5 bis 180° erfolgen. Das eingestrahlte Licht ist erfindungsgemäß vorzugsweise linear polarisiert, jedoch kann auch elliptisch polarisiertes, zirkular polarisiertes oder auch unpolarisiertes Licht verwendet werden.

Bei der transcutanen Messung von Streuintensitäten, d.h. bei der Messung durch Gewebeschichten und Blutgefäße hindurch, liegt ein Teil der Streustrahlung als inkohärente Strahlung vor. Dieser Streustrahlungsanteil wird zwar durch Sekundärstreuung an anderen Schichten stark geschwächt, ist jedoch grundsätzlich nicht vermeidbar. Die inkohärente Streuung kann durch Filter (z.B. Polarisationsfilter) teilweise unterdrückt werden, wenn die Primärstrahlung polarisiert ist. Anderenfalls muß sie als Untergrund vom Signal abgezogen werden.

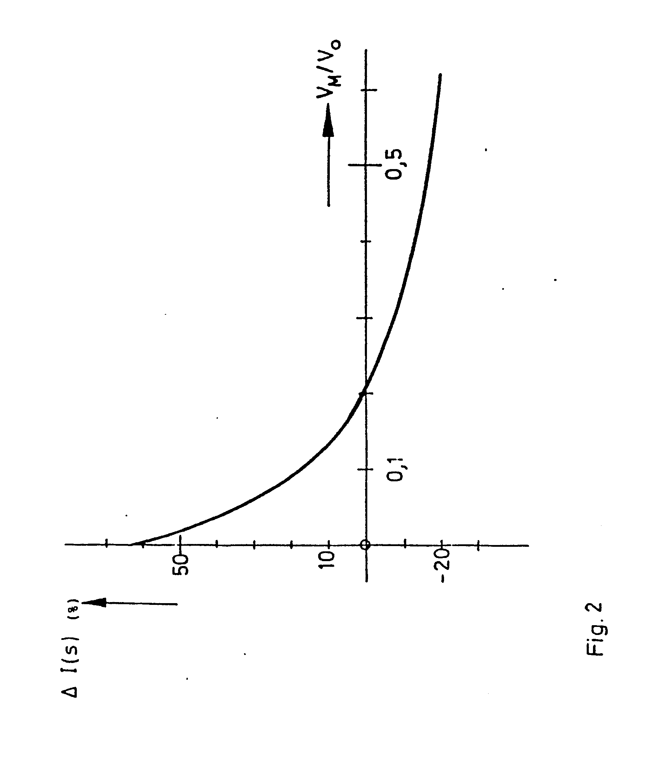

Eine Abschätzung der theoretisch zu erwartenden Änderung der Intensität der Streustrahlung bei einer Änderung der Konzentration einer Komponente ergibt sich aus den Berechnungen von G. Fournet (A. Guinier, X-Ray Diffraction, W. H. Freeman and Company, San Francisco, 1963). Auf dieser Basis ergibt sich für die Abhängigkeit der Intensitätsänderung vom Verhältnis $V_M/V_o$ das in Fig. 2 dargestellte Bild. An der Ordinate ist die prozentuale Änderung der Streuintensität, bezogen auf die Intensität bei s = 0, und an der Abszisse das Verhältnis der Volumina angetragen. Mit steigender Glucosekonzentration, d.h. fallendem $V_M/V_o$, kann im Streuwinkelbereich um 1° Prinzipiell sowohl eine zunehmende als auch eine abnehmende Signalamplitude erreicht werden, was auch von der Polarität der Teilkomponenten etwa bei einer Differenzbildung abhängt. Außerhalb des Kleinwinkelstreubereichs (z.B. bei θ > 10°) ist jedoch entsprechend Gleichung (1) grundsätzlich eine Zunahme der Intensität der Streustrahlung mit der Konzentration einer niedermolekularen Substanz, z.B. von Glucose, zu erwarten.

Die Änderung des freien Volumens insbesondere der Proteine und des Hämoglobins im Fall des Blutes durch eine physiologisch auftretende Änderung der Glucosekonzentration, z.B. bei Diabetikern, von beispielsweise 5 mmol/l auf 15 mmol/l führt zu einer Änderung von $V_M/V_o$ um etwa 0,4 im Bereich von 0,2 bis 0,6. Damit ist in erster Näherung eine Intensitätsänderung von 20 % zu erwarten, was ein recht beachtlicher Wert ist. Voraussetzung hierfür ist, daß im Winkelbereich um 1° gemessen wird, was eine sehr gute Meßtechnik erfordert. Bei einer gröberen Meßtechnik daher mit einer wesentlich verringerten Meßempfindlichkeit gerechnet werden.

Eine zusätzliche grundsätzliche Schwierigkeit bei der Messung z.B. von Blutkomponenten durch Laser-Lichtstreuung an einem geeigneten Körperteil, z.B. dem Ohrläppchen, ergibt sich aus dessen wechselnder Durchblutung, was dazu führen kann, daß gänzlich verschiedene Meßsignale bei gleicher Glucosekonzentration erhalten werden. Eine entsprechende Korrektur ist jedoch erfindungsgemäß technisch ohne weiteres möglich. Hierfür wird vorteilhafterweise die Intensität des Zentralstrahls oder das Verhältnis der Intensität des Zentralstrahls zur Intensität der Streustrahlung herangezogen. So wird beispielsweise bei einer fest vorgegebenen Lichtintensität die Intensität des Zentralstrahls gemessen und durch Differenzbildung mit einem individuell vorgebbaren Sollwert eine Korrektur des Meßsignals herbeigeführt, wobei ein empirisch zu bestimmender Vorfaktor benützt werden kann.

Die Messung der Konzentration einer Komponente einer mehrkomponentigen Lösung, z.B. der Glucosekonzentration im Blut, erfolgt erfindungsgemäß, wie oben erläutert und abgeleitet, durch Analyse der Streukurvenform und Ermittlung desjenigen Winkelbereichs der Streustrahlung, in dem die größte Intensitätsänderung in Abhängigkeit von der Konzentration der zu bestimmenden Komponente vorliegt. Am einfachsten kann die Messung erfindungsgemäß nach dem sog. Zweisignalverfahren durchgeführt werden, jedoch ist selbstverständlich auch eine Intensitätsmessung unter kontinuierlicher Änderung des Streuwinkels durchführbar, was jedoch apparativ einen größeren Aufwand erfordert. Beim Zweisignalverfahren wird die Intensität des Zentralstrahls sowie eine Streulichtintensität unter einem bestimmten Streuwinkel gemessen. Der Winkelbereich, innerhalb dessen die Streulichtintensität gemessen wird, wird erfindungsgemäß vorteilhaft so gewählt, daß die Änderung der Intensität der Streustrahlung mit der Änderung der zu messenden Konzentration in diesem Bereich maximal wird.

Die erfindungsgemäße Vorrichtung umfaßt prinzipiell einen optischen Teil, einen elektrischen bzw. elektronischen Teil sowie einen mechanischeh Teil. Sie weist im wesentlichen folgende Bestandteile auf:

A) Optischer Teil:
a) Lichtquelle (Laser, Laserdiode)
b) gegebenenfalls einen Umlenkspiegel (fest oder beweglich)
c) Probe bzw Probenhalter + Küvette
d) gegebenenfalls elnen Umlenkspiegel (fest oder beweglich)
e) optisches Empfangsteil mit getrennter Erfassung von mindestens zwei Winkelbereichen, falls die Spiegel b) und/oder d) nicht beweglich sind,
und
f) ein oder mehrere Lichtdetektoren.

B) Elektrischer Teil:
a) Stromversorgung für die Lichtguelle, gegebenenfalls steuerbar oder regelbar
b) Stromversorgung für die Lichtdetektoren
c) gegebenenfalls Intensitätsregelung für die Lichtquelle
d) Verstärker für die Signale der Lichtdetektoren der verschiedenen Winkelbereiche, entsprechend der Sektorierung des optischen Empfangsteils
e) Differenzbildner, Addierglied, Quotientenbildner und/oder Multiplizierer
f) Signalverarbeitungsteil (Mikroprozessor, Mikrocomputer oder Rechner) mit Speicher, Konstanten, Ein- und Ausgabemöglichkeit
g) Signalanzeigegerät
h) Ausgabevorrichtung (Drucker, Schreiber udgl.).

Der mechanische Aufbau hängt vom jeweils vorgesehenen Verwendungszweck ab; der mechanische Teil ist erfindungsgemäß insbesondere als klapp- oder schiebbarer Meßaufbau ausgebildet, der eine feste oder bewegliche Lichtquelle, feste oder bewegliche Umlenkeinrichtungen wie Umlenkspiegel, ein festes oder bewegliches Lichtempfangsteil, vorzugsweise mit Winkelbereichssektorierung, sowie eine Befestigungseinrichtung zur Befestigung an cinem geeigneten Körperteil, z.B. am Ohrläppchen, aufweist.

Zur Messung am Ohr eignet sich besonders eine ohrclipartige Ausführungsform, wobei die erfindungsgemäße Vorrichtung zugleich in Form von üblichem Schmuck augebildet sien kann.

Die einzelnen optischen und elektrischen bzw. elektronischen Komponenten können dabei erfindungsgemäß auf unterschiedliche Vorrichtungteile verteilt und mit diesen teilweise oder ganz integriert sein. Erfindungsgemäß ist vorzugsweise der elektronische Teil teilweise oder ganz in integrierter Form ausgebildet; vorteilhafterweise kann auch der optische Teil in einer integrierten Form ausgebildet sein.

Für die Komponenten des optischen Sendeteils können handelsübliche Bauteile verwendet werden.

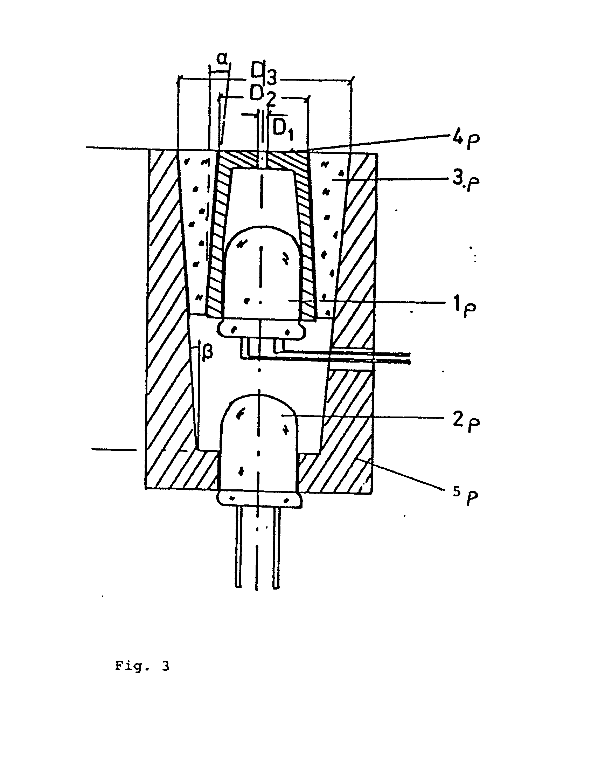

Das optische Empfangsteil, das mindestens zwei Winkelbereiche getrennt voneinander erfaßt, besitzt vorzugsweise einen Aufbau, der im Prinzip der in Fig. 3 dargestellten Ausführungsform entspricht. Die in Fig. 3 dargestellte Vorrichtung ist gekennzeichnet durch ein Gehäuse 5p, in dessen Bodenfläche ein Lichtdetektor 2p zentrisch vorgesehen ist, und dessen Innenfläche sich vom Lichtdetektor 2p aus gesehen nach außen konisch erweitert, einen an der offenen Seite des Gehäuses 5p konzentrisch X der Konusfläche vorgesehenen ringförmigen Glaskörper 3p, der aus Glas oder einem anderen-lichtdurchlässigen Material besteht und dessen Innenfläche sich vom Lichtdetektor 2p aus gesehen nach außen konisch verengt, eine im Glaskörper 3p konzentrisch angeordnete strahlungsundurchlässige oder strahlungsundurchlässig beschichtete Hülse 4p, die an ihrem nach außen gerichteten Ende bis auf eine zentrale Öffnung verschlossen ist, und einen in der Hülse 4p konzentrisch und in der optischen Achse der Anordnung vorgesehenen Lichtdetektor 1p, wobei der Durchmesser D2 der zentralen Öffnung der Hülse 4p, der Durchmesser D2 der außen liegenden Bodenfläche der Hülse 4p und der Durchmesser D3 der außen liegenden Bodenfläche des Glaskörpers 3p sowie der Konus-Halbwinkel α der Hülse 4p und der Konus-Halbwinkel β des Gehäuses 5p bzw. des Innenkonus des Glaskörpers 3p so ausgelegt sind, daß die gesamte in den zugrundegelegten Streuwinkelbereich fallende Streustrahlung vom Lichtdetektor 2p erfaßt wird.

Die Innenfläche des Gehäuses 5p bzw. die Außenfläche des Glaskörpers 3p und/oder die Außenfläche der Hülse 4p bzw die Innenfläche des Glaskörpers 3p können auch vorteilhafterweise im Querschnitt parabelförmig gekrümmt ausgebildet sein.

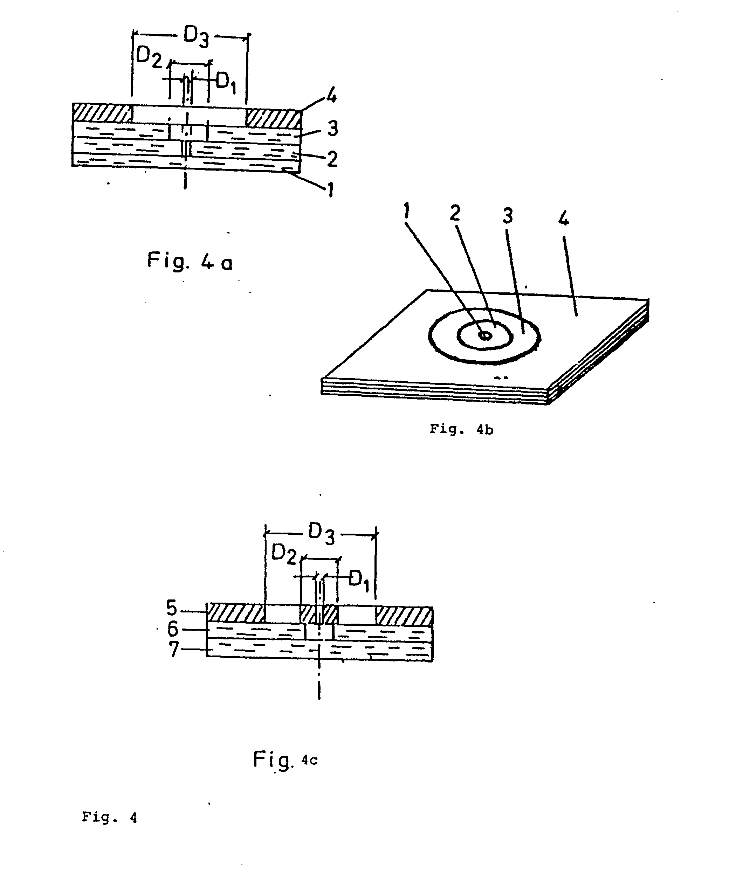

In Fig. 4 ist eine weitere Ausführungsform der erfindungsgemäßen Vorrichtung dargestellt, bei der der optische Empfangsteil flach ausgebildet ist. In Fig.4a ist eine sandwichartige Anordnung von vier Schichten dargestellt, von denen die unterste Schicht 1 eine lichtempfindliche Schicht ist; die Schichten 2, 3 und 4 können entweder lichtempfindlich sein oder lediglich Maskenfunktion besitzen, je nachdem, ob nach einem Zweisignalverfahren, einem Dreisignalverfahren oder einem Mehrsignalverfahren gemessen werden soll. Die in Fig.4a dargestellte Variante des optischen Empfangsteils ist vorzugsweise so ausgebildet, daß die unterste Schicht 1 lichtempfindlich ist, die darüberliegende zweite Schicht 2 strahlungsundurchlässig ist und eine in der optischen Achse liegende zentrale Öffnung mit dem Durchmesser D1 aufweist, die darüberliegende dritte Schicht 3 lichtempfindlich ist und eine konzentrische zentrale Öffnung mit dem Durchmesser D2 aufweist, und die darüberliegende vierte Schicht 4 eine zentrale konzentrische Öffnung mit dem Durchmesser D3 besitzt, die als Blendenmaske wirkt.

In Fig. 4b ist die resultierende Struktur schematisch dargestellt.

Eine weitere erfindungsgemäße Ausführungsform zeigt Fig. 4c. Bei dieser Variante des ebenfalls flach ausgebildeten optischen Empfangsteils ist eine oberste Schicht 5 aus einem lichtundurchlässigen Material vorgesehen, die eine ringförmige Öffnung mit den Durchmessern D2 und D3 und eine zentrale Öffnung mit dem Durchmesser D1 aufweist und als Maske für die darunterliegenden Schichten 6 und 7 wirkt. Die unter der obersten Schicht 5 vorgesehene Schicht 6 ist lichtempfindlich und weist eine zentrale konzentrische Öffnung auf, deren Durchmesser zwischen D1 und D2 liegt; unter ihr befindet sich eine ebenfalls lichtempfindliche Schicht 7. Diese Anordnung eignet sich speziell für das Zweisignalverfahren.

Die Durchmesser D2 und D3 werden in den Fällen der Vorrichtungen der Fig. 4a und 4c so gewählt, daß der vorgesehene Streuwinkelbereich erfaßt wird.

Gegenüber der in Fig. 3 dargestellten Ausführungsform des optischen Empfangsteils haben die Vorrichtungen der Fig. 4a und 4c den Vorteil einer erheblich geringeren Einbautiefe; diese flachen Empfangsteile können jedoch nicht mit handelsüblichen Bauelementen aufgebaut werden.

Der elektrische bzw elektronische Teil der erfindungsgemäßen Vorrichtung kann aus handelsüblichen Bauelementen aufgebaut sein. Derjenige Teil, der zur Intensitätsregelung der Lichtquelle vorgesehen ist, kann erforderlichenfalls ein Regler mit einer speziellen Form der Regelkennlinie sein, da beispielsweise Laserdioden bekanntermaßen eine stark nichtlineare Strom-Intensitäts-Charakteristik aufweisen.

Die Signalverarbeitung kann erfindungsgemäß sowohl digital als auch analog vorgenommen werden.

Digitale Signalverarbeitung

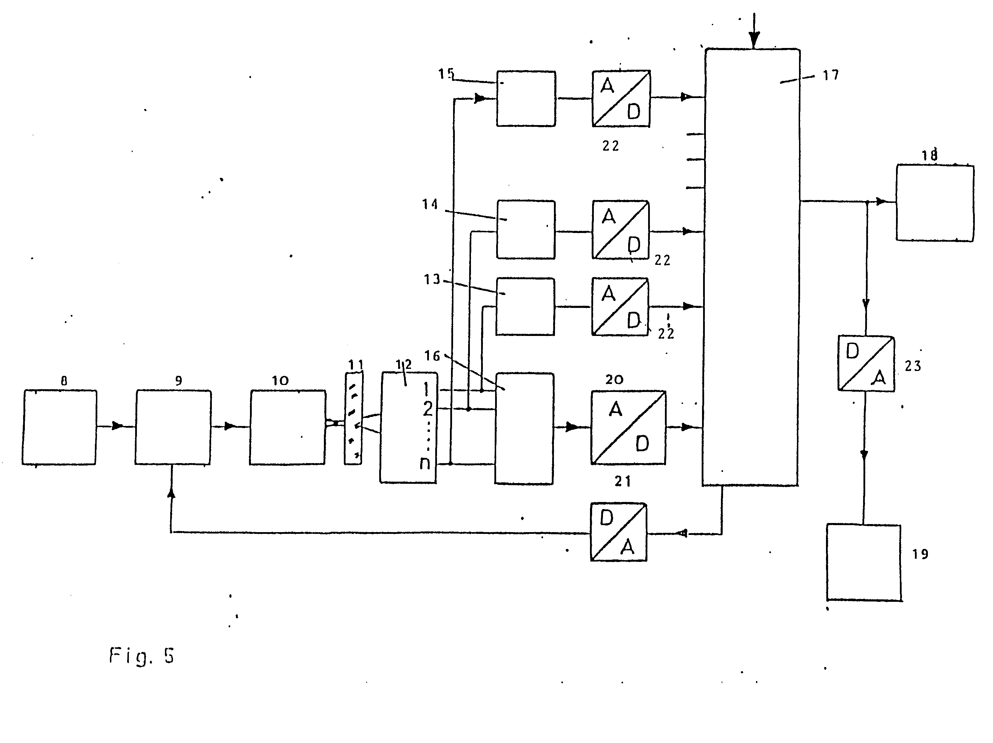

In Fig. 5 ist ein Prinzipschaltbild des elektrischen Teils einer erfindungsgemäßen Vorrichtung mit digitaler Signalverarbeitung dargestellt. Die Anordnung umfaßt eine Stromversorgung 8, einen Regelverstärker 9 für die Lichtquelle, eine Lichtquelle 10 sowie verschiedene Lichtdetektoren 12, die das von der Probe 11 durchgelassene bzw.gestreute Licht erfassen und entsprechende Meßsignale liefern. Im einfachsten Fall sind nur zwei Lichtdetektoren 12 vorgesehen, aus deren Meßsignalen in Verbindung mit einem Referenzsignal bis zu maximal fünf Signalgrößen (zwei absolute und drei relative) abgeleitet werden können. Die Erzeugung der relativen Signalgrößen kann dabei vor oder nach der Umwandlung der Meßsignale in digitale Signale erfolgen.

Die Meßsignale der Lichtdetektoren 12 werden durch zugeordnete Signalverstärker 13 bis 15 verstärkt und über Analog-Digital-Wandler 22 zu einem Meßwertverarbeitungsteil 17 geleitet, das aus einem Mikroprozessor, einem Mikrocomputer oder einem sonstigen Rechner bestehen kann. Ein oder mehrere Differenzverstärker 16 oder dergleichen, liefern die relativen Intensitäten. Das Meßwertverarbeitungsteil erzeugt aufgrund vorgegebener Funktionen bzw. Programme aus den Meßsignalensausgangssignale, welche die Signalausgabe steuern, korrigiert die Meßsignale durch Vergleich mit eingegebenen Konstanten und/ oder in anderer Weise, beispielsweise durch Differenzbildung, Quotientenbildung, Addition oder Subtraktion, und erzeugt ferner die erforderliche Regelgröße, die dann z.B. über DIA-Wandler 21 zum Regelverstärker 9 gelangt. Das Meßwertverarbeitungsteil 17 ist ferner gegebenenfalls so ausgebildet, daß es steigende oder fallende Tendenzen des Meßsignals bzw. des ausgegebenen Signals erkennt und anzeigt; das Meßwertverarbeitungsteil erzeugt ferner vorzugsweise durch eine geeignete Peripherie akustische und/oder optische Signale bei einstellbaren oberen und/oder unteren Grenzwerten, beispielsweise Warnsignale. Die Vorrichtung kann weiter so ausgelegt sein, daß sie mit anderen Datenverarbeitungseinrichtungen kompatibel ist und an andere Datenverarbeitungseinrichtungen angeschlossen werden kann. Die Ergebnisausgabe erfolgt über eine Anzeige einrichtung 18, beispielsweise ein Digitalvoltmeter die Vorrichtung umfaßt ferner eine Ausgabeeinrichtung 19, die einen Meßwertdrucker, einen Schreiber oder ein ähnliches registrierendes und/oder anzeigendes Instrument darstellt und über einen DIA-Wandler 23 am Meßwertverarbeitungsteil 17 angeschlossen ist.

Analoge Signalverarbeitung

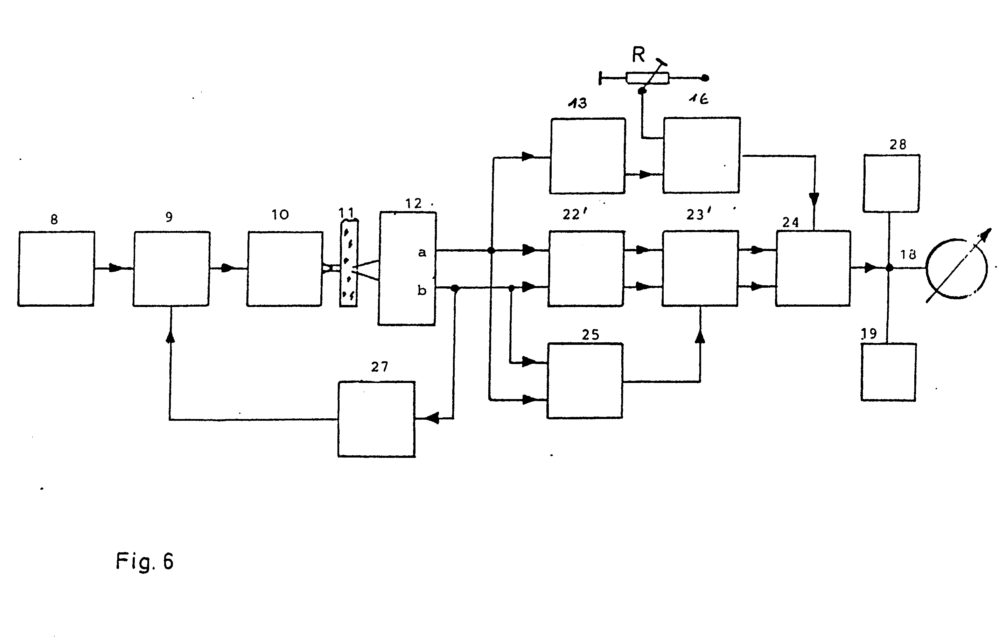

In Fig. 6 ist ein Blockschaltbild einer erfindungsgemäßen Ausführungsform des elektrischen Teils dargestellt, bei der die Signalverarbeitung analog erfolgt. Die Schaltung umfaßt wie die in Fig. 5 dargestellte Schaltung

**0 074 428**

eine Stromversorgung 8, einen Regelverstärker 9 für die Lichtquelle, eine Lichtquelle 10 und zwei Lichtdetektoren 12, die den Zentralstrahl bzw.die von der Probe 11 kommende Streustrahlung erfassen.

Die in Fig. 6 dargestellte Schaltung ist für das Zweisignalverfahren ausgelegt; Verfahren, die auf der Erfassung von drei oder mehr Signalen beruhen,führen bei analoger Signalverarbeitung rasch zu sehr umfangchend nur zwei Lichtdetektoren 12 auf.

Die Vorrichtung umfaßt ferner einen Differenzverstärker mit Integrator 22', der das Differenzsignal der Signale a und b der Lichtdetektoren 12 integriert (Zeitkonstante $\tau$ = 1 bis 10 s); das resultierende Signal wird in den Verstärkern 23' und 24 nochmals verstärkt und mit einer Anzeigeeinrichtung 18 angezeigt. Der Verstärker 23' wird dabei durch das Verhältnis der beiden Meßwerte a/b über den Quotientenbildner 25 und der Verstärker 24 durch die Differenz des durch den Verstärker 13 verstärkten Meßsignals a und der am Widerstand R einstellbaren Spannung die durch einen Differenzverstärker 16 gebildet wird, gesteuert. Das Meßsignal wird durch die in Fig. 6 dargestellte Schaltung entsprechend durch den Verstärker 23' relativ und durch den Verstärker 24 absolut korrigiert; das resultierende Signal wird mit der Anzeigeeinrichtung 18 angezeigt.

Die Absolutintensität des Signals b kann ferner erforderlichenfalls über einen Regelverstärker 27 zur Stabilisierung der Intensität der Lichtquelle 10 herangezogen werden. Die Schaltung von Fig.6 umfaßt ferner einen Grenzwertgeber 28, der einen oberen und/oder unteren Grenzwert des Signals signalisiert; (er besteht beispielsweise aus zwei Schmitt-Triggern). Die steigende oder fallende Tendenz des Signals kann vom Integrator 22' abgeleitet werden. Die Schaltung umfaßt schließlich auch eine Ausgabeeinrichtung 19, d.h. beispielsweise ein registrierendes und gegebenenfalls auch anzeigendes Meßinstrument, z.B. einen Drucker oder einen Schreiber.

Auch die Schaltung von Fig. 6 kann ferner so ausgelegt sein, daß sie mit anderen, gegebenenfalls unabhängigen Datenverarbeitungseinrichtungen oder -anlagen kompatibel ist und verbunden werden kann.

Die in den Fig. 5 und 6 dargestellten Schaltungen können ferner auch so ausgelegt sein, daß mit Hilfe der resultierenden Signale, die von der Anzeigeeinrichtung 18 angezeigt oder von der Ausgabeeinrichtung 19 registriert werden, unabhängige Geräte wie beispielsweise Infusionseinrichtungen und insbesondere Vorrichtungen zur Insulindosierung, die gegebenenfalls auch implantierbar sein können, angesteuert werden können.

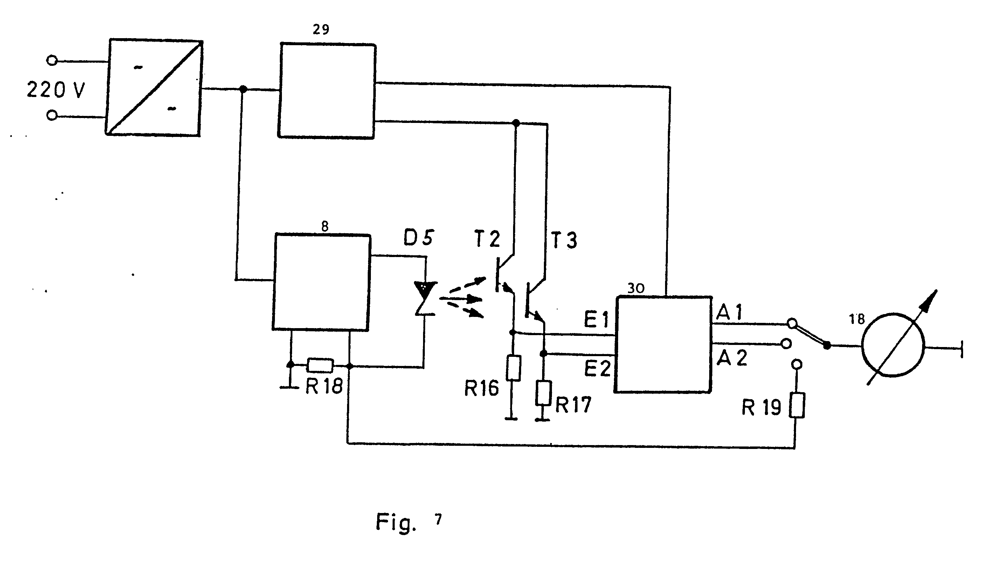

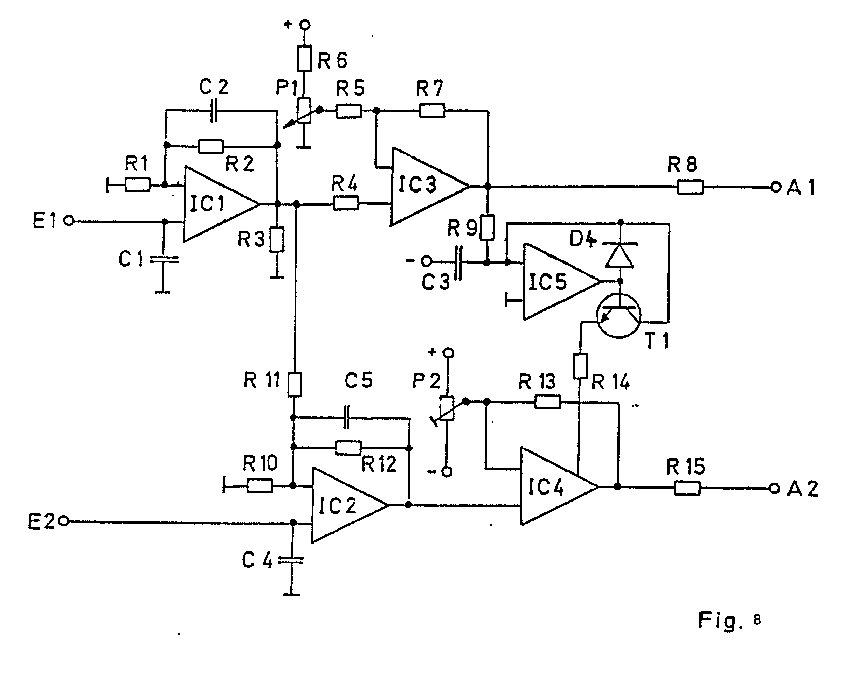

In Fig. 7 ist das Prinzipschaltbild einer technischen Ausführungsform der erfindungsgemäßen Vorrichtung dargestellt, die auf der analogen Signalverarbeitung beruht. Auch in dieser Ausführungsform sind alle Funktionen der Schaltung von Fig. 6 mit Ausnahme des Regelverstärkers 27, des Grenzwertgebers 28 und der Ausgabeeinrichtung 19 enthalten. Die Funktionen sind jedoch teilweise vereinfacht bzw. zusammengefaßt, wobei der Meßverstärker 30, der bei Fig. 8 näher erläutert ist, die Funktionen des Signalverstärkers 13, des Integrators 22', der Verstärker 23 und 24, des Quotientenbildners 25 und des Differenzverstärkers 16 von Fig. 6 übernimmt.

Zur Stromversorgung wird von der Netzspannung, (z.B. 220 V) ausgegangen, die zunächst in mehrere, beispielsweise vier Niederspannungen umgesetzt wird. Eine alternative Stromversorgung durch Batterien kann vorgesehen sein. Die Schaltung weist eine Stromversorgung 29 auf, die den Meßverstärker 30 mit einer stabilisierten Spannung, beispielsweise ± 6 V, versorgt. Ferner werden die beiden Lichtdetektoren T2 und T3 getrennt davon durch die Stromversorgung 29 mit einer hochstabilisierten Spannung, zB +30 V, versorgt. Die Widerstände R16 und R17 sind die Signalwiderstände der Lichtdetektoren T2 und T3. Die CW-Laserdiode D5 als Lichtquelle wird von der Stromversorgung 8 mit einer elektronisch geregelten, ebenfalls hochstabilisierten Spannung versorgt, wobei die Stromversorgung 8 insbesondere so ausgelegt ist, daß sie auch Spannungsspitzen der Netzspannung in jedem Fall von der Diode fernhält. Der Strom der Laserdiode D5, der über den Bypass-Widerstand R18 fließt, kann mit Hilfe des Serienwiderstands R19 erforderlichenfalls durch die Anzeigeeinrichtung 18 angezeigt und überwacht werden. Der Meßverstärker 30 arbeitet sowohl als Differenzverstärker als auch als Einzelverstärker, wie im folgenden näher erläutert ist.

Der Meßverstärker 30 erhält an seinen Eingängen E1 und E2 die entsprechenden Signale der Lichtdetektoren T2 bzw. T3 zugeführt. Er besitzt die Ausgänge A1 und A2, die mit der Anzeigeeinrichtung 18 verbunden werden können.

In Fig. 8 ist der Meßverstärker 30 im einzelnen schematisch dargestellt. Er bildet aus den an den Eingängen E1 und E2 ankommenden Signalen über IC1 und IC2 mit Hilfe des Widerstands R11 die Differenz der Eingangssignale. Diese Differenz wird über IC4 und R15 zum Ausgang A2 geleitet. Das an E1 ankommende Eingangssignal wird ferner durch IC1 und IC3 auch direkt verstärkt und über R8 zum Ausgang A1 geführt.

Die Verstärkung des Signalkanals für das Differenzsignal, also des Zweiges E2-IC2-IC4-R15, wird in Abhängigkeit von der Spannung an E1 geregelt. Die Wirksamkeit dieser Regelung wird durch die Widerstände R9 und R14 sowie durch das Potentiometer P2 bestimmt, das den Arbeitspunkt des Operationsverstärkers IC4 festlegt. Mit IC5 wird über den Transistor T1, die Diode D4 und den Widerstand R14 die Spannung am Ausgang von IC3 in einen Steuerstrom für den in seiner Verstärkung regelbaren Operationsverstärker IC4 umgewandelt. Das Potentiometer P1 dient zur Sollwerteinstellung für diese Steuerung, mit der sich beispielsweise bei der transcutanen Blutglucosebestimmung am Ohrläppchen eine Durchblutungskorrektur einstellen läßt.

Die maximale Verstärkung im Signalzweig beträgt mindestens 10000, für die Verstärkung des Zweigs E1 - A1 ist eine Verstärkung von etwa 1000 ausreichend. Dieser Faktor ist aber naturgemäß auch noch von der Empfindlichkeit des Anzeigeinstruments abhängig.

Die Arbeitspunkte der Operationsverstärker werden durch die folgenden Widerstände bestimmt, wobei

9

diese auch die Verstärkung bestimmen: bei IC 1 von Rl und R2, bei IC 3 von R5, R6 und R7, bei IC 2 von R10 und R12, bei IC 4 außer von P 2 auch von R13. Außerdem sind die üblichen, in der Figur nicht wiedergegebenen Offset-Null-Potentiometer und Kondensatoren zur Freguenzkompensation vorgesehen. Die Kondensatoren C1 und C4 dienen zur Senkung der Eingangsstörspannungen. Die Kondensatoren C2, C3 und C5 verringern die Schwingneigung des Verstärkers und dienen gleichzeitig zur Integration des jeweiligen Signals.

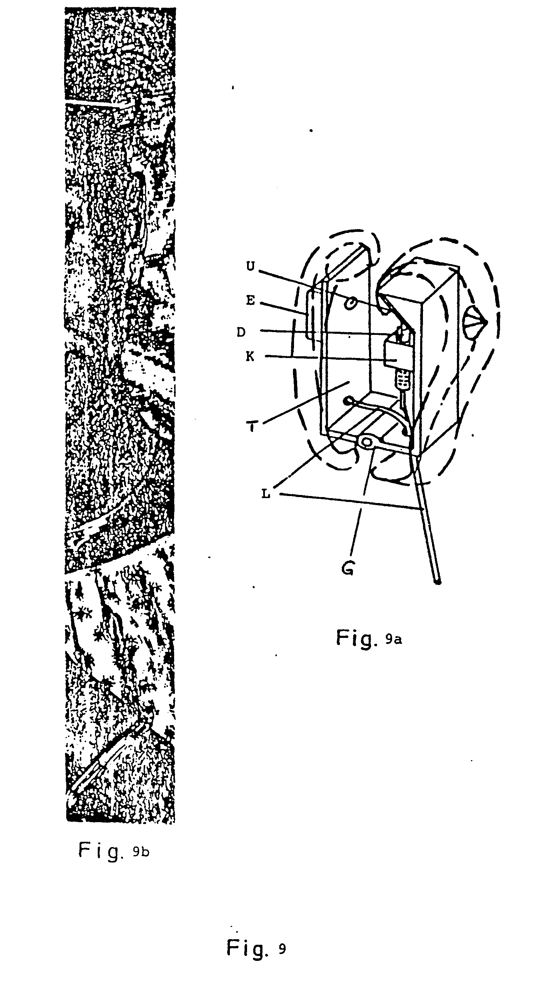

In Fig. 9 ist eine zur transcutanen Blutglucosebestimmung in vivo am Ohrläppchen vorgesehene erfindungsgemäße Vorrichtung dargestellt, wobei Fig. 9a den prinzipiellen Aufbau der Vorrichtung und Fig. 9b eine entsprechende Realisierung einer am Körper zu tragenden Vorrichtung zeigen.

Der Meßaufbau ist mit durchgezogenen Strichen dargestellt; die gestrichelten Linien entsprechen dekorativen Elementen.

Die Vorrichtung ist als Ohrclip ausgeführt, wobei die Laserdiode D in einem Kühlblock K vorgesehen ist, der jedoch nicht zwingend erforderlich ist. Die Vorrichtung weist ferner einen Umlenkspiegel U auf, der das von der Laserdiode emittierte Licht um etwa 90° umlenkt, so daß es durch das in den Ohrclip eingeführte Ohrläppchen hindurchgeht; der optische Empfangsteil E empfängt das durch das Ohrläppchen hindurchgehende bzw. gestreute Laserlicht, wobei bis zu 4 oder auch mehr Signale getrennt voneinander erfaßt werden können. Das optische Empfangsteil E ist in der oben erläuterten Sandwichtechnik oder etwa in Form der in Fig. 3 dargestellten Lichtpfeifenanordnung (vgl. Light-pipe cone, in: D.E. Williamson, Cone Channel Condensor Optics, J. Opt. Soc. Amer. 42 (1952) 712) ausgebildet und entsprechend zur Unterscheidung der verschiedenen Winkelbereiche der Strahlung sektoriert. Das optische Empfangsteil E und der Teil der Vorrichtung mit der Lichtquelle weisen ferner entsprechende elektrische Zuleitungen bzw. Verbindungsleitungen zur Stromquelle und gegebenenfalls zur Signalverarbeitungseinrichtung auf. Der optische Teil ist in einem U-förmigen Träger T vorgesehen, der ein federndes Gelenk G im Bodenbereich aufweist, zwischen dessen Seitenteile ein Ohrläppchen eingeführt werden kann.

Die äußere Gestaltung der in Fig. 9a dargestellten erfindungsgemäßen Vorrichtung kann frei gewählt werden und wird lediglich von Designüberlegungen bestimmt, so daß sie dem individuellen Geschmack des Trägers der Vorrichtung angepaßt werden kann.

Das in Fig. 9a gestrichelt angedeutete Design der Vorrichtung ist in Fig. 9b anhand einer Photographie einer konkreten Ausführungsform abgebildet. Die Vorrichtung wird ähnlich wie bei einem üblichen Ohrclip durch einen einfachen Klappmechanismus am Ohrläppchen befestigt, wobei die Vorrichtung vorzugsweise möglichst leicht und klein ausgeführt ist. Die Stromversorgung für die Lichtquelle sowie die Signalverarbeitungseinrichtung werden vorzugsweise unabhängig von der ohrclipartigen eigentlichen Meßeinrichtung vorgesehen und beispielsweise an einer geeigneten Stelle, z.B. in einer Tasche oder unter der Kleidung, getragen. Teile der erfindungsgemäßen Vorrichtung, z.B. die Ausgabeeinrichtung 19 oder die Anzeigeeinrichtung 18, können ferner beispielsweise in Form einer Armbanduhr oder armbanduhrartigen Vorrichtung oder etwa einer halskettenartigen Vorrichtung ähnlich einer Umhängeuhr ausgestaltet sein.

Mathematische Struktur der Meßdaten und ihre Korrektur

Der zu erwartende Verlauf der Signalamplitude in Abhängigkeit von der Konzentration der zu bestimmenden Substanz, beispielsweise Glucose, entspricht im Experiment nicht vollständig dem in Fig. 2 dargestellten theoretischen Signalverlauf, da die Signalamplitude auch von der Dimensionierung und den Eigenschaften der Bauelemente der jeweiligen Vorrichtung abhängt. So resultiert beispielsweise bei einer erfindungsgemäßen Vorrichtung, die etwa Fig. 9 entspricht, ein der Glucosekonzentration zuzuordnender Streuwinkeibereich von etwa 10° gegenüber dem theoretischen Idealwert (mit maximaler Empfindlichkeit) von etwa 1,5°. Untersucht man unter diesen Gegebenheiten den grundsätzlichen Verlauf der Signalamplitude in Abhängigkeit von der Intensität des Zentralstrahls, so kann eine Potenzfunktion etwa der Form

$$\triangle I(s) = a \cdot I_{(o)}^{b} \quad (7)$$

mit $I_{(o)}$ = Intensität des Zentralstrahls nach Durchgang durch die Probe

angesetzt werden. Dieser Ansatz gilt zunächst für jede beliebige Probe. Die Probeneigenschaften werden durch die Konstanten a und b berücksichtigt. Während die Konstante a überwiegend durch die Transmissionseigenschaften beeinflußt wird, werden durch die Konstante b vor allem die Struktureinflüsse und bei biologischen proben die Konzentrationen der Teilkomponenten charakterisiert.

Eine Papierprobe mit einer solchen Dicke, daß die resultierende mittlere Intensität gleich der einer biologischen Probe ist (Papierdicke etwa 0,15 mm), führt zu einem Wert für die Konstante a von 0,19 und für die Konstante b von 1,15. Dies bedeutet einen nahezu linearen Zusammenhang zwischen $\triangle I$ und $I_{(o)}$ (vgl Gleichung (7)), wie dies für eine 'physikalische' Probe auch zu erwarten ist. Im Vergleich dazu ergibt sich z.B. bei der Messung am Ohrläppchen im Mittel ein Wert für a von 0,003 und für b von 1,5.

Für die Auswertung von transcutanen Messungen der Blutglucosekonzentration in vivo eignet sich folglich ein mathematischer Ansatz einfachster Form wie z.B.

$$b = d + e \cdot S \text{ bzw } S = \frac{b-d}{e} \quad (8),$$

wobei die Konstanten d und e empirisch bestimmt werden können. S bedeutet die Glucosekonzentration. Meßgrößen sind $\triangle I(s)$ und $I_{(o)}$.

Die Konstanten a und b ergeben sich aus einer leicht durchführbaren Kurvenanpassung mit Wertepaaren der oben genannten Meßgrößen bei Messung beimehreren Intensitäten. Die Konstanten d und e sind im Prinzip für jede Probe einmal zu bestimmen; überraschenderweise hat sich jedoch ergeben, daß sie von Probe zu Probe nur wenig verschieden sind.

Die Meßwertkorrektur bei der Messung biologischer Proben, z.B. im Hinblick auf die Korrektur wegen wechselnder Durchblutung des Ohrläppchens, muß berücksichtigen, daß sich die effektiven Probendicke x ändert. Sie kann deshalb auch nicht einfach durch Verhältnisbildung vorgenommen werden, wie dies z.B. bei üblichen optischen Zweistrahlverfahren möglich ist. Die Verhältnisbildung liefert zwar Unabhängigkeit von der Absolutintensität, jedoch keinen Ausgleich für veränderliche Probendicke. Die Korrekturfunktion z.B. zur Korrektur unterschiedlicher Durchblutung muß im allgemeinen eine exponentielle Form haben.

Für die Signalbildung ist andererseits erfindungsgemäß stets eine Differenzbildung notwendig, da sich nur so eine Aussage über die Form der Streukurve mit einfachen Mitteln ergibt.

Zur Herleitung der Korrekturfunktion kann wie folgt vorgegangen werden:.

Die Abhängigkeit der Lichtintensität beim Durchstrahlen eines absorbierenden Mediums ist in bekannter Weise durch das Absorptionsgesetz

$$I = I_0 . e^{-m.x}$$

gegeben, wobei

x die Probendicke und

m den Absorptionskoeffizienten

bedeuten.

Der Absorptionskoeffizient m ist definiert als $\frac{2\pi k}{\lambda 2}$

mit $k = \sqrt{n^2 - \varepsilon . \mu}$,

wobei

$\lambda$ die Wellenlänge des verwendeten Lichts,

$\varepsilon$ die Dielektrizitätskonstante und

$\mu$ die Permeabilität

bedeuten.

Ferner gilt

$$n^2 = 1 + 4\pi.\alpha.N,$$

wobei $\alpha$ die Polarisierbarkeit und

N die Anzahl der streuenden Teilchen pro ml bedeuten.

Bei Durchblutungsänderungen ändert sich naturgemäß auch die Anzahl der streuenden Teilchen pro ml:

Wenn ein Blutgefäß mit einer Lineardimension $x_0$ (z.B. dem Durchmesser) angenommen wird, das sich in einem Abstand $x_1$ zum nächsten Blutgefäß befindet, gilt für das Volumen 'ohne Blut':

$$V_0 \sim x_1^3;$$

für das Volumen "mit Blut' gilt

$$V_1 \sim (x_1 + x_0)^3.$$

Daraus folgt für das Verhältnis der Volumina

$$V_1/V_0 = \frac{(x_1 + x_0)^3}{x_1^3} \text{ bzw. } V_1 = V_0 \left(1 + 3\tfrac{x_0}{x_1} + 3\left(\tfrac{x_0}{x_1}\right)^2 + \left(\tfrac{x_0}{x_1}\right)^3\right) \quad (10).$$

Das Volumen, in dem sich Blut befindet, ergibt sich also zu

$$V = V_1 - V_0 = V_0 \left(3\tfrac{x_0}{x_1} + 3\left(\tfrac{x_0}{x_1}\right)^2 + \left(\tfrac{x_0}{x_1}\right)^3\right) \quad (11)$$

im Bereich

$$0 \leq x_0 \leq x_1.$$

Untersucht man das Verhältnis $x_0/x_1$, wie es im Mittel im Gewebe beispielsweise des Ohrläppchens vorliegt, ergibt sich ein Wert von etwa 0,5 bis 0,8. In diesem Bereich ändert sich das Volumen, in dem sich Blut befindet, in guter Näherung quadratisch, wie aus Gleichung (11) folgt. Somit resultiert für die Anzahl der streuenden Teilchen näherungsweise

$$N \sim (x_0/x_1)^2$$

und daraus mit Gleichung (9) die Beziehung

$$k \sim x_0/x_1 \quad (12).$$

Als Probendicke x in Gleichung (9) ist ebenfalls $x_0$ einzusetzen, so daß sich insgesamt die Beziehung

$$I = I_0 . e^{\beta} x_0^2 \quad (13)$$

ergibt, in der ß einen entsprechenden Koeffizienten bedeutet.

Zur mathematischen Berechnung muß Gleichung (13) differenziert werden, wobei sich der einfache Zusammenhang

$$d I \sim x_0 \quad (14)$$

ergibt, da $x_0$ selbst sehr klein ist, und sich die Exponentialfunktion infolge der nur kleinen Änderungen ebenfalls nur wenig ändert.

Durch Umkehrung von Gleichung (14) wird die an sich unbekannte Größe $x_0$ aus der Intensitätsänderung erhalten

$$x_0 = \gamma(I - I_0') \quad (15),$$

wobei $I_0'$ die Intensität bei normaler Durchblutung bedeutet.

Daraus folgt als Korrekturfunktion

$$\Delta I' = \Delta I(s) . e^{-\gamma(I - I_0')^2} \quad (16).$$

Gegenüber der normalen Durchblutung kann, sowohl eine stärkere als auch eine schwächere Durchblutung vorliegen, so daß die Korrektur sowohl positiv als auch negativ sein kann; das Vorzeichen der Korrektur muß entsprechend bestimmt werden. Dies geschieht am besten wiederum mit der bereits benutzten Größe I - Io', so daß sich als endgültige Korrekturformel ergibt:

$$\Delta I = \Delta I(s) \cdot (1 + \delta \cdot (I-I_o') \cdot e^{-\gamma(I-I_o')^2}) \quad (17).$$

Die Konstanten $\delta$, $\gamma$ und $I_0'$ sind experimentell bestimmbar; in einfachen Fällen können sie auch theoretisch ermittelt werden, da ihre physikalischen Bedeutungen klar definiert sind.

Aus den Meßdaten, also z.B. aus den Spannungen an E1 und E2 in Fig. 7, kann die Meßgröße, z.B. die Glucosekonzentration, auf drei verschiedene Weisen ermittelt werden, und zwar:

1. Aus Gleichung (8), wobei die Konstante b aus Gleichung (7) mit E1 = $\Delta$ I(s) und E2 = $I_{(0)}$, unter Verwendung eines Mittelwerts für a, errechnet wird;

2. aus Gleichung (7) mit einem oder mehreren Wertepaaren (E1; E2) zur Ermittlung der Konstanten a und b nach der "Best-fit'-Methode und durch iterative Angleichung von S nach Gleichung (8);

3. durch Messung von $_{E1}$ und $_{E2}$ bei einer festen Bezugsintensität $I_0$ und unter Verwendung der Mittelwerte der Konstanten $\underline{a}$ und $\underline{b}$ und $\Delta$ I(s) aus Gleichung (7), wobei E2 = $I_{(0)}$ gesetzt wird. Dann kann durch einen linearen Ansatz (oder in anderen Fällen durch einen anderen mathematischen Zusammenhang) die direkte Beziehung zwischen der Meßgröße und der Signalgröße hergestellt werden.

Dieser Zusammenhang kann beispielsweise die Form

S = K . $\Delta$ I(s) - A (18)

besitzen, in der

S die Glucosekonzentration

und

A und K Konstante

bedeuten.

Alle drei Korrekturen sollten zum gleichen Ergebnis führen. Da jedoch die Meßdaten auch bei sorgfältiger Meßweise bei biologischen Proben naturgemäß besonderen Schwankungen unterworfen sind, ist es in vielen Fällen zweckmäßig, alle drei Auswertungsverfahren parallel zu verwenden und ihre Ergebnisse zu kombinieren, um so zu einer von Einzelmeßfehlern weitgehend unabhängigen Meßwertanzeige zu gelangen. Am einfachsten geschieht dies durch eine geeignete Mittelwertbildung.

Eine einfache und zugleich sehr günstige Möglichkeit der Korrektur der Meßsignale für unterschiedliche Durchblutung bei transcutanen Messungen in vivo, insbesondere am Ohrläppchen, besteht in der Berücksichtigung der Hauttemperatur an der Meßstelle, die in einer relativ direkten Beziehung zur Gewebedurchblutung an der betreffenden Meßstelle steht. Durch eine entsprechende mathematische Korrekturfunktion, die z.B. empirisch bestimmt werden kann, können die Meßsignale aufgrund der gemessenen Hauttemperatur auf einen bestimmten Durchblutungszustand hin korrigiert werden. Die erfindungsgemäße Vorrichtung weist entsprechend günstigerweise einen Temperaturfühler zur Messung der Hauttemperatur an bzw. in der Nähe der Meßstelle auf, dessen Signal zur Korrektur der Meßsignale in der Signalverarbeitungseinrichtung herangezogen wird.

Für diese Korrektur nach der Hauttemperatur ist es erforderlich, daß sich die Meßvorrichtung bei allen Messungen unverändert an einer bestimmten Meßstelle befindet bzw. in reproduzierbarer Weise angebracht werden kann.

Eine derartige Festlegung ist insbesondere bei weiblichen Anwendern des in Fig. 9 dargestellten Ohrclips, die für Schmuckzwecke durchstochene Ohrläppchen besitzen, in besonders einfacher Weise möglich, wenn die Meßvvorrichtung in das Loch im Ohrläppchen eingesteckt wird.

Die erfindungsgemäße Vorrichtung gemäß dem Prinzip von Fig. 9 weist daher günstigerweise für solche Anwendungsfälle einen entsprechenden Dorn auf, der durch das Loch im Ohrläppchen durchgesteckt wird.

Die oben erläuterten mathematischen Operationen sind beispielsweise auf üblichen programmierbaren Taschenrechnern durchführbar. Selbstverständlich können zur Auswertung auch Mikroprozessoren bzw. andere Rechner und Rechenanlagen herangezogen werden. Ein Programm in einer "Low level'-Programmiersprache benötigt zur Kombination der drei Verfahren samt Mittelwertbildung etwa 220 Programmschritte.

## Beispiel

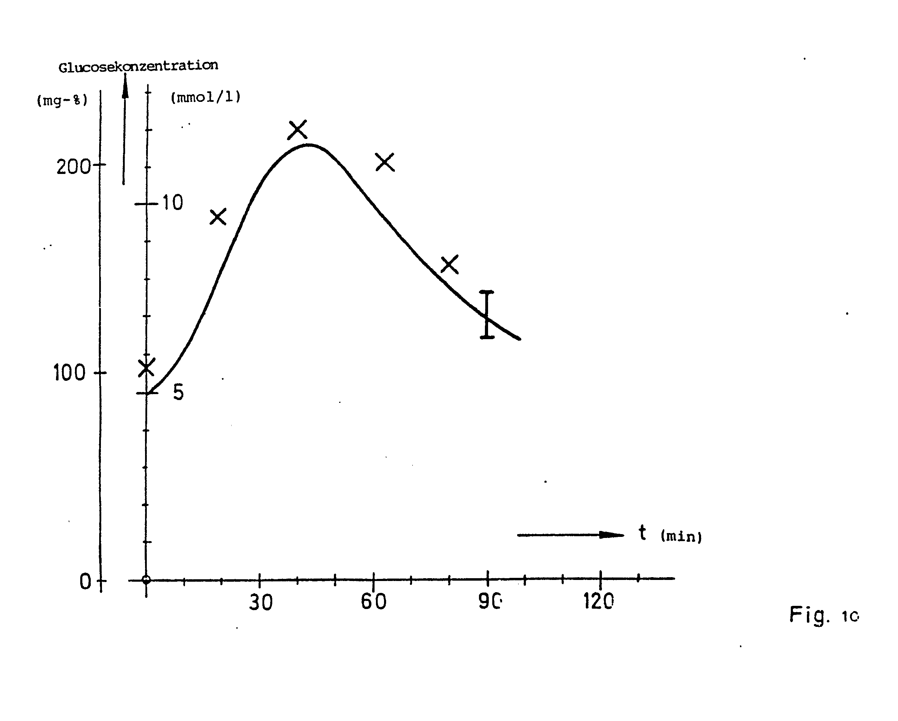

In Fig. 10 sind experimentelle Ergebnisse einer mit einer erfindungsgemäßen Vorrichtung durchgeführten kontinuierlichen transcutanen Blutglucosemessung dargestellt, wobei eine Vorrichtung ähnlich der von Fig. 9 verwendet wurde. Die Messungen wurden an einer gesunden Versuchsperson durchgeführt, der zu Versuchsbeginn zur Erzeugung einer Glucosebelastung oral 400 ml Dextro O.G.-T. verabreicht wurden. Dextro O.G.-T.-Lösung zur oralen Glucosebelastung entspricht einer physiologischen Menge von 100 g wasserfreier Glucose. In 400 ml Saft sind 17 g wasserfreie Glucose, 13 g Maltose, 11 g Maltotriose und 53 g höhere Oligosaccharide enthalten. Dieses Gemisch entspricht nach Hydrolyse im Darmtrakt der oben angegebenen Menge Glucose (BM Toleranz-Test, Boehringer Mannheim GmbH, 6800 Mannheim 31).

Die in Form eines Ohrclips ausgeführte Vorrichtung wurde am Ohrläppchen der Versuchsperson angebracht. Die Vorrichtung arbeitete nach dem Zweisignalverfahren, wobei die Absolutintensität des Zentralstrahls zur Korrektur herangezogen wurde. Das resultierende Differenzsignal der beiden Meßsignale wurde durch die Signalverarbeitungsvorrichtung erzeugt und mit einem Schreiber aufgezeichnet.

Zu Vergleichszwecken wurde die Differenz der beiden Meßsignale ohne vorherige Korrektur ebenfalls aufgezeichnet. Hierbei ergab sich, daß die erfindungsgemäß durchgeführte Korrektur zur Erzielung quantitativer Meßergebnisse erforderlich ist.

Alle 20 min wurden der Versuchsperson Blutproben entnommen, die routinemäßig nach dem in der klinischen Analytik üblichen Hexokinaseverfahren mit einem Autoanalyzer auf den Gehalt an Blutglucose untersucht wurden. Die mit dem Autoanalyzer nach dem herkömmlichen Verfahren ermittelten Meßergebnisse sind als Kreuze in Fig. 10 eingetragen. Von dieser Methodik ist bekannt, daß sie im Vergleich mit anderen herkömmlichen Methoden die höchsten Werte liefert, wie etwa aus H. Förster und M. Haslbeck, Diagnostik 5 (1972) 311 - 315, hervorgeht. Die mit der erfindungsgemäßen Vorrichtung kontinuierlich erhaltenen Meßergebnisse sind als durchgezogene Linie dargestellt. Die Ergebnisse zeigen, daß das erfindungsgemäße Verfahren und die erfindungsgemäße Vorrichtung zu einer quantitativen Blutglucosebestimmung geeignet sind, da ausgezeichnete Übereinstimmung der erhaltenen Meßergebnisse mit den Vergleichswerten besteht.

Zusätzlich zu diesen Messungen wurden kontinuierliche Messungen nach dem erfindungsgemäßen Verfahren an einer kranken Versuchsperson (Diabetiker) durchgeführt, bei der ferner laufend Blutglucosebestimmungen nach der oben angegebenen herkömmlichen Verfahrensweise durchgeführt wurden. Da der Ohrclip von Messung zu Messung jeweils verändert angebracht bzw. wegen der Abnahme zwischen den Messungen nicht in völlig reproduzierbarer Weise angelegt werden kann, läßt sich durch derartige Messungen, bei den mit Sicherheit bei jeder Messung jeweils eine andere Durchstrahlungsstelle erfaßt wird, der entsprechende Korrelationskoeffizient ermitteln. Bei 10 derartigen Messungen wurde ein Korrelationskoeffizient von r = 0,4 erreicht, was einer ausgezeichneten Reproduzierbarkeit der Messungen auch bei jeweils geänderter Durchstrahlungsstelle entspricht.

Das erfindungsgemäße Verfahren sowie die erfindungsgemäße Vorrichtung eignen sich aufgrund ihres zugrundeliegenden technischen Konzepts in besonderem Maße für eine Miniaturisierung, die noch um Größenordnungen über die in Fig. 9 dargestellte Ausführungsform hinausgehen kann, wobei auch Mikrominiaturisierung und folglich auch die Möglichkeiten der Konzeption implantierbarer Vorrichtungen. bereits heute technisch durchaus realistisch sind.

Das erfindungsgemäße Verfahren sowie die erfindungsgemäße Vorrichtung sind nicht auf die quantitative Bestimmung von Glucose in wäßrigen und insbesondere biologischen Systemen beschränkt, da sich bei geeigneter Auswahl der Streuwinkelbereiche auch andere gelöste niedermolekulare und auch hochmolekulare Substanzen in einfachen und zusammengesetzten Systemen in prinzipiell gleicher Weise quantitativ bestimmen lassen.

Die erfindungsgemäße Vorrichtung kann ferner aufgrund des zugrundeliegenden allgemeinen technischen Konzepts in sehr weitem Maße im Rahmen des Erfindungsgedankens modifiziert werden.

Das erfindungsgemäße Verfahren sowie die erfindungsgemäße Vorrichtung stellen somit eine völlig neuartige Alternative zu bisherigen Verfahren und Vorrichtungen zur Blutglucosebestimmung wie auch zur transcutanen Bestimmung anderer Blutkomponenten und Blutwerte (Erythrocytenzahl, Hb-Wert udgl.) dar.

**Patentansprüche**

1. Verfahren zur quantitativen Bestimmung der Konzentration gelöster niedermolekularer und hochmolekularer Substanzen sowie von Blutkörperchen bzw. Blutzellen in Mehrkomponentensystemen, wobei sich die Einzelkomponenten in den Lineardimensionen der Moleküle, ihrer Struktur und/oder Zusammensetzung und/oder in der Molekülmasse hinreichend unterscheiden, durch

(a) Lichtstreuung an der Probe und Analyse der Streukurvenform unter Ermittlung mindestens eines Streuwinkelbereichs pro zu bestimmende Komponente, in dem die stärkste Abhängigkeit der Intensität der Streustrahlung von der Konzentration der jeweiligen Komponente vorliegt,

(b) Korrektur der gemessenen Intensität der jeweiligen Streustrahlung mit der gemessenen Intensität des Zentralstrahls und

(c) Zuordnung der korrigierten Meßgröße zur Konzentration der zu bestimmenden Komponente.

2. Verfahren nach Anspruch 1, dadurch gekennzeichnet, daß die Intensität der Streustrahlung unter kontinuierlicher Änderung des Streuwinkels gemessen wird.

3. Verfahren nach Anspruch 1, dadurch gekennzeichnet, daß die Intensität der Streustrahlung unter einem einzigen vorgegebenen Streuwinkel bzw.in einem einzigen vorgegebenen Streuwinkelbereich gemessen wird.

4. Verfahren nach Anspruch 1, dadurch gekennzeichnet, daß die Intensität der Streustrahlung bei mehreren

**0 074 428**

diskreten vorgegebenen Streuwinkeln bzw. in mehreren Streuwinkelbereichen gemessen wird und gegebenenfalls bei entsprechender Auswertung mehrere Komponenten einer vielkomponentigen Lösung gleichzeitig bestimmt werden.

5. Verfahren nach einem der Ansprüche 1 bis 4, dadurch gekennzeichnet, daß das Meßsignal bei sich verändernder effektiver Probendicke, etwa zur Durchblutungskorrektur, korrigiert wird, wobei die Korrekturgröße aus der gemessenen Intensität des Zentralstrahls hergeleitet wird.

6. Verfahren nach einem der Ansprüche 1 bis 5, dadurch gekennzeichnet, daß die Korrektur des Meßsignals digital so vorgenommen wird, daß ihre Art durch eine mathematische Funktion festgelegt und ihre Größe durch Eingabe von Konstanten vorgegeben wird.

7. Verfahren nach einem der Ansprüche 1 bis 5, dadurch gekennzeichnet, daß die Korrektur des Meßsignals analog durch eine geeignete Verstärkungssteuerung und durch Vergleich mit einem einstellbaren Sollwert, wie etwa dem Spannungswert eines Potentiometers, durchgeführt wird.

8. Verfahren nach einem der Ansprüche 1 bis 7, dadurch gekennzeichnet, daß eine Lichtquelle mit linear, elliptisch oder zirkular polarisierter Strahlung verwendet wird.

9. Verfahren nach einem der Ansprüche 1 bis 8, dadurch gekennzeichnet, daß die Signalverarbeitung in den Schritten (b) und (c) analog durchgeführt wird.

10. Verfahren nach einem der Ansprüche 1 bis 9, dadurch gekennzeichnet, daß der Meßwert der Streustrahlung nach Korrektur mit Hilfe der Intensität des Zentralstrahls nach einer oder mehreren Relativintensitäten korrigiert wird.

11. Verfahren nach einem der Ansprüche 1 bis 9, dadurch gekennzeichnet, daß nach einem Zwei- oder Mehrsignalverfahren gearbeitet wird, und die gemessene Intensität der Streustrahlung durch Differenzwertbildung zu einem Referenzwert nach einer oder mehreren Relativintensitäten und/oder Produkten von Intensitäten korrigiert wird.

12. Verfahren nach einem der Ansprüche 1 bis 11, dadurch gekennzeichnet, daß die Meßwerte vor oder nach der Korrektur gemittelt werden.

13. Verfahren nach einem der Ansprüche 1 bis 12, dadurch gekennzeichnet, daß zur Messung der Intensität der Streustrahlung und des Zentralstrahls nur ein einziger Lichtdetektor verwendet wird, wobei der Winkel der Lichtquelle gegenüber der Probe durch Bewegung der Lichtquelle und/oder durch bewegliche Umlenkspiegel verändert wird.

14. Verfahren nach einem der Ansprüche 1 bis 13, dadurch gekennzeichnet, daß zwischen Probe und Lichtdetektor(en) Filter eingeschaltet werden, welche die Meßempfindlichkeit verbessern und die Streustörstrahlung verringern.

15. Vorrichtung zur Durchführung des Verfahrens nach einem der Ansprüche 1 bis 14
mit
- einer Lichtquelle,
- einem Probenteil,
- einem oder mehreren Lichtdetektoren, welche die Intensität des Zentralstrahls und der Streustrahlung in mindestens einem vorgegebenen Winkelbereich erfassen,
- einer elektronischen Signalverarbeitungseinrichtung zur Korrektur der Meßsignale,
- einer Stromversorgung
sowie
- einer Signalanzeigeeinrichtung oder einer Signalausgabevorrichtung,
gekennzeichnet durch
- einen im Prinzip etwa U-förmigen Träger (T) mit einem in seinem Bodenbereich vorgesehenen Gelenk (G) mit einer Feder, die die beiden Seitenteile des Trägers unter einem Federdruck gegeneinander drückt, und einem derartigen Abstand der beiden Seitenteile voneinander, daß in den Zwischenraum zwischen den beiden Seitenteilen des Trägers (T) ein menschliches Ohrläppchen eingeführt und darin festgehalten werden kann,
- eine an bzw. in einem Seitenteil des Trägers (T) vorgesehene, gegebenenfalls in einem Kühlblock (K) angeordnete Laserdiode (D) als Lichtquelle,
- einen über der Laserdiode (D) angebrachten Umlenkspiegel (U), der das von der Laserdiode (D) emittierte Licht um etwa 90° in etwa waagerechte Richtung umlenkt,
und
- ein gegenüber dem Umlenkspiegel (U) am anderen Seitenteil des Trägers vorgesehenes optisches Empfangsteil (E) mit mindestens einem Lichtdetektor, das die Intensität des Zentralstrahls und der Streustrahlung des durch das in dem Träger (T) eingeführte Ohrläppchen hindurchgehenden bzw. gestreuten Lichts der Laserdiode (D) in mindestens einem vorgegebenen Winkelbereich erfaßt und entsprechende elektrische Signale liefert,
- wobei die elektronische Signalverarbeitungseinrichtung die Signalanzeigeeinrichtung und/oder die Signalausgabeeinrichtung sowie die Stromversorgung jeweils mit dem Träger (T) integriert oder von ihm unabhängig vorgesehen sind.

16. Vorrichtung nach Anspruch 15, gekennzeichnet durch ein optisches Empfangsteil (E), das so ausgebildet ist, daß es durch Sektorierung zwei Winkelbereiche getrennt erfaßt.

17. Vorrichtung nach Anspruch 15 oder 16, dadurch gekennzeichnet, daß das optische Empfangsteil (E) in Form einer Lichtpfeife ausgebildet ist.

18. Vorrichtung nach einem der Ansprüche 15 bis 17, gekennzeichnet durch ein optisches Empfangsteil (E)

14

mit

einem Gehäuse (5p), in dessen Bodenfläche ein Lichtdetektor (2p) zentrisch vorgesehen ist, und dessen Innenfläche sich vom Lichtdetektor (Zp) aus gesehen nach außen konisch erweitert,

einem an der offenen Seite des Gehäuses (5p) konzentrisch in der Konusfläche vorgesehenen ringförmigen Glaskörper (3p) aus Glas oder einem anderen lichtdurchlässigen Material, dessen Innenfläche sich vom Lichtdetektor (2p) aus gesehen nach außen konisch verengt,

einer im Glaskörper (3p) konzentrisch angeordneten strahlungsundurchlässigen oder strahlungsundurchlässig beschichteten Hülse (4p), die an ihrem nach außen gerichteten Ende bis auf eine zentrale öffnung verschlossen ist,

und einem in der Hülse (4p) konzentrisch und in der optischen Achse der Anordnung vorgesehenen Lichtdetektor (Ip),

wobei der Durchmesser (D1) der Zentralöffnung der Hülse (4p), der Durchmesser (D2) der außen liegenden Bodenfläche der Hülse (4p) und der Durchmesser (D3) der außen liegenden Bodenfläche des Glaskörpers (3p) sowie der Konus-Halbwinkel ($\alpha$) der Hülse (4p) und der Konus-Halbwinkel (F) des Gehäuses (5p) bzw. des Innenkonus des Glaskörpers (3p) so ausgelegt sind, daß die in den zugrundeliegenden Streuwinkelbereich fallende Streustrahlung vom Lichtdetektor (2p) möglichst vollständig erfaßt wird.

19. Vorrichtung nach Anspruch 18, dadurch gekennzeichnet, daß die Innenfläche des Gehäuses (5p) bzw. die Außenfläche des Glaskörpers (3p) und/oder die Außenfläche der Hülse (4p) bzw. die Innenfläche des Glaskörpers (3p) im Querschnitt parabelförmig gekrümmt sind.

20. Vorrichtung nach Anspruch 15 oder 16, gekennzeichnet durch ein flach ausgebildetes optisches Empfangsteil, das aus vier übereinander angeordneten Schichten (1 bis 4) aufgebaut ist, wobei die unterste Schicht (1) lichtempfindlich ist, die darüberliegende zweite Schicht (2) strahlungsundurchlässig ist und eine in der optischen Achse liegende zentrale Öffnung mit dem Durchmesser (DI) aufweist, die darüberliegende dritte Schicht (3) lichtempfindlich ist und eine zentrale konzentrische Öffnung mit dem Durchmesser (D2) aufweist, und die darüberliegende vierte Schicht (4) eine konzentrische zentrale Öffnung mit dem Durchmesser (D3) besitzt, wobei die Durchmesser (D1 bzw D2 und D3) so gewählt sind, daß die Schicht (1) den Zentralstrahl und die Schicht (3) die in den der Messung zugrundeliegenden Streuwinkelbereich fallende Streustrahlung möglichst vollständig erfassen.

21. Vorrichtung nach Anspruch 15 oder 16, gekennzeichnet durch ein flach ausgebildetes optisches Empfangsteil, das aus drei übereinander angeordneten Schichten (5 bis 7) aufgebaut ist, wobei die oberste Schicht (5) lichtundurchlässig ist und eine ringförmige Öffnung mit den Durchmessern (D2 und D3) und eine zentrale Öffnung mit dem Durchmesser (D1) aufweist und unter der obersten Schicht (5) eine lichtempfindliche Schicht (6) mit einer zentralen konzentrischen Öffnung, deren Durchmesser zwischen (DI und D2) liegt, und eine darunterliegende lichtempfindliche Schicht (7) vorgesehen sind, wobei die Durchmesser (DI bzw. D2 und D3) so gewählt sind, daß die Schicht (7) den Zentralstrahl und die Schicht (6) die in den der Messung zugrundeliegenden Streuwinkelbereich fallende Streustrahlung möglichst vollständig erfassen.

22. Vorrichtung nach einem der Ansprüche 15 bis 21, gekennzeichnet durch
- einen Regelverstärker (9) für die Lichtquelle (10),
- einen oder mehrere Differenzverstärker (16), die aus Meßsignalen der Lichtdetektoren (12) der relativen Intensität des zugeordneten Streulichts entsprechende Signale liefern, die in einem A/D-Wandler (20) in entsprechende Digitalsignale umgewandelt werden,
- A/D-Wandler (22), welche die Meßsignale der Lichtdetektoren (12), von denen einer die Intensität des Zentralstrahls und die anderen die Intensität der von der Probe (11) ausgehenden Streustrahlung erfassen, nach Verstärkung in Signalverstärkern (13, 14, 15) in entsprechende Digitalsignale umwandeln, und
- ein Meßwertverarbeitungsteil (17), das aufgrund vorgegebener Funktionen bzw. Programme an den Digitalsignalen der Lichtdetektoren (12) bzw des bzw.der Differenzverstärker (16) Ausgangssignale für eine Anzeigeeinrichtung (18) bzw. für eine Ausgabeeinrichtung (19) mit vorgeschaltetem D/A-Wandler (23) sowie ferner eine Regelgröße erzeugt, die nach Umwandlung in ein Analogsignal in einem D/A-Wandler (21) zum Regelverstärker (9) geführt ist.

23. Vorrichtung nach einem der Ansprüche 15 bis 21, gekennzeichnet durch
- einen Regelverstärker (9) für die Lichtquelle (10),
- einen Differenzverstärker mit Integrator (22), der das Differenzsignal der Meßsignale (a, b) von zwei Lichtdetektoren (12), von denen einer die Intensität des Zentralstrahls und der andere die Intensität der von der Probe (11) ausgehenden Streustrahlung erfaßt, integriert,
- zwei hintereinandergeschaltete, steuerbare Verstärker (23, 24), die das Ausgangssignal des Differenzverstärkers mit Integrator (22) weiter verstärken,
- einen Quotientenbildner (2S), der das Verhältnis (a/b) der beiden Meßsignale (a, b) bildet und mit einem entsprechenden Ausgangssignal den Verstärker (23) steuert,
- einen Verstärker (13), der das Meßsignal (a) verstärkt,
sowie
- einen Differenzverstärker (16), der das Ausgangssignal des Verstärkers (13) mit einer vorgegebenen Bezugsspannung vergleicht, und dessen Ausgangssignal den Verstärker (24) steuert.

24. Vorrichtung nach einem der Ansprüche 15 bis 23, gekennzeichnet durch eine Laserdiode als Lichtquelle.

25. Vorrichtung nach einem der Ansprüche 15 bis 24, gekennzeichnet durch Phototransistoren als Lichtdetektoren zur Messung der Intensität des Zentralstrahls und der Streustrahlung.

26. Vorrichtung nach einem der Ansprüche 15 bis 25, gekennzeichnet durch einen Mikrocomputer oder Mikroprozessor als bzw. im Signalverarbeitungsteil.

27. Vorrichtung nach einem der Ansprüche 15 bis 26, gekennzeichnet durch einen Schreiber, einen Plotter oder einen Kleindrucker, gegebenenfalls zusammen mit einem Taschenrechner, als Ausgabeeinrichtung (19).

28. Vorrichtung nach einem der Ansprüche 15 bis 27, gekennzeichnet durch eine Einrichtung, die bei Erreichen eines vorgegebenen oberen und/oder unteren Grenzwerts des Meßwerts ein akustisches, optisches und/oder élektrisches Signal auslöst.

29. Vorrichtung nach einem der Ansprüche 15 bis 28, dadurch gekennzeichnet, daß sie mikrominiaturisiert ist.

30. Vorrichtung nach einem der Ansprüche 15 bis 29, dadurch gekennzeichnet, daß sie zur Steuerung einer Infusionseinrichtung ausgelegt ist.

31. Vorrichtung nach Anspruch 30, dadurch gekennzeichnet, daß sie zur Steuerung einer Insulin-Infusionseinrichtung ausgelegt ist.

32. Vorrichtung nach einem der Ansprüche 15 bis 31, gekennzeichnet durch einen Temperatur-Meßfühler zur Messung der Hauttemperatur und eine Einrichtung zur Korrektur des Meßsignals nach einer vorgegebenen Korrekturfunktion aufgrund der ermittelten Hauttemperatur.

## Claims

1. A process for the quantitative determination of the concentration of dissolved low molecular weight and high molecular weight substances, and of blood particles or blood cells in multi-component systems, in which the individual components sufficiently differ in the linear dimensions of their molecules, in structure, and/or in composition and/or in molecular mass,

by

(a) light scattering at the sample and analysis of the scattering curve configuration and determination of at least one scattering angle range per component to be detected, in which there is maximum dependence of the intensity of the scattered radiation on the concentration of the respective component,

(b) correction of the measured intensity of the respective scattered radiation with the measured intensity of the central beam, and

(c) attribution of the corrected measured value to the concentration of the component to be determined.

2. Method according to claim 1, characterized in that the intensity of the scattered radiation is measured while the scattering angle is continuously varied.

3. Method according to claim 1, characterized in that the intensity of the scattered radiation is measured at a single predetermined scattering angle or in a single predetermined scattering angle range.

4. Method according to claim 1, characterized in that the intensity of the scattered radiation is measured at a plurality of discrete predetermined scattering angles, or in a plurality of scattering angle ranges, and optionally, upon appropriate evaluation, several components of a multi-component solution are determined simultaneously.

5. Method according to one of claims 1 to 4, characterized in that the measured signal is corrected upon varying actual sample thickness, e.g. for blood flow correction, and the corrective quantity is derived from the measured intensity of the central beam.

6. Method according to one of claims 1 to 5, characterized in that the correction of the measured signal is effected digitally such that the mode thereof is determined by a mathematical function and its quantity is predetermined by the input of constants.

7. Method according to one of claims 1 to 5, characterized in that the correction of the measured signal is effected analogously by suitable amplification control and by comparison with an adjustable rated value, e.g. the voltage level of a potentiometer.

8. Method according to one of claims 1 to 7, characterized in that a light source with linearly, elliptically, or circularly polarized radiation is employed.

9. Method according to one of claims 1 to 8, characterized in that signal processing is performed analogously in steps (b) and (c).

10. Method according to one of claims 1 to 8, characterized in that the measured value of the scattered radiation after correction is corrected by means of the intensity of the central beam according to one or more relative intensities.

11. Method according to one of claims 1 to 8, characterized in that a two-signal or multi-signal technique is adopted, and the measured intensity of the scattered radiation is corrected by forming the difference to obtain a reference value according to one or more relative intensities and/or products of intensities.

12. Method according to one of claims 1 to 11, characterized in that the measured values are averaged prior to or after correction.

13. Method according to one of claims 1 to 12, characterized in that for measurement of the intensity of the scattered radiation and of the central beam only a single light detector is used, and the angle of the light source relative to the sample is varied by movement of the light source and/or by movable deflecting mirrors.

14. Method according to one of claims 1 to 13, characterized in that between sample and light detector(s) filters are interposed which improve the measuring accuracy and suppress parasitic scattered radiation.

15. Apparatus for carrying out the method according to one of claims 1 to 14, comprising
- a light source,
- a sample member,
- one or more light detectors detecting the intensity of the central beam and of the scattered radiation in at least one predetermined angular range,
- an electronic signal processing means for correction of the measured signals,
- a power supply,
and
- a signal display means or signal output means,
characterized by
- a substantially U-shaped support (T) with a joint (G) provided in its bottom region and with a spring urging the two side portions of the support towards each other under spring pressure, and with the side portions spaced apart a distance to allow the insertion of a human earlobe into the space between the two side portions of the support (T) to be held therebetween,
- a laser diode (D) provided as light source at or in a side portion of the support (T) and optionally arranged in a cooling block (K),
- a deflecting mirror (U) mounted above the laser -diode (D) and deflecting the light emitted by the laser diode (D) by about 80o to substantially horizontal direction,
and
- an optical receiving unit (E) provided opposite the deflecting mirror (U) at the other side portion of the support and comprising at least one light detector detecting the intensity of the central beam and of the scattered radiation of the light from the laser diode (D) beamed or scattered, respectively, through the earlobe introduced into the support (T) in at least one predetermined angular range and providing corresponding electric signals,
- said electronic signal processing means, signal display means, and/or signal output means and the power supply being integrated with the support (T) or provided independently thereof.

16. Apparatus according to claim 15, characterized by an optical receiving unit (E) which is so designed that it separately covers two angular ranges by sectoring.

17. Apparatus according to claims 15 or 16, characterized in that the optical receiving unit (E) is designed as an optical pipe.

18. Apparatus according to one of claims 15 to 17, characterized by an optical receiving unit (E) comprising
a housing (5p) in the bottom face of which a light detector (2p) is centrically disposed and whose inner surface tapers conically outwardly, when seen from the light detector (2p),
an annular glass member (3p) made of glass or another light-transmissive material and provided concentrically in the cone surface on the open side of the housing (5p) so that the inner surface of said member tapers conically outwardly, when seen from the light detector (2p),
a sleeve (4p) which is radiation impermeable or radiation-impermeably coated and arranged concentrically in the glass member (3p) and closed at its outwardly facing end, except for a central opening,
and a light detector (1p) disposed concentrically in the sleeve (4p) in the optical axis of the system,
the diameter (D1) of the central opening in the sleeve (4p), the diameter (D2) of the externally disposed bottom face of the sleeve (4p), and the diameter (D3) of the externally disposed bottom face of the glass member (3p) and the cone half-angle ($\alpha$) of the sleeve (4p) and the cone half-angle ($\beta$) of the housing (5p), or of the inner cone of the glass member (3p), respectively, being so selected that the scattered radiation arriving in the underlying scattering angle range is substantially completely detected by the light detector (2p).

19. Apparatus according to claim 18, characterized in that the inner surface of the housing (5p) or the outer surface of the glass member (3p), respectively, and/or the outer surface of the sleeve (4p) or the inner surface of the glass member (3p), respectively, have a parabolically curved cross section.

20. Apparatus according to claims 15 or 16, characterized by a flat optical receiving unit composed of four superposed layers (1 to 4), the lowermost layer (1) being photosensitive, the overlying second layer (2) being radiation-impermeable and having a central opening of the diameter (D1) disposed in the optical axis, the overlying third layer (3) being photosensitive and having a central concentric opening of the diameter (D2), and the overlying fourth layer (4) having a concentric central opening of the diameter (D3), the diameters (D1, D2, D3, respectively) being so selected that the layer (1) substantially completely detects the central beam, and the layer (3) substantially completely detects the scattered radiation arriving in the scattering angle range underlying the measurement.

21. Apparatus according to claims 15 or 16, characterized by a flat optical receiving unit composed of three superposed layers (5 to 7), with the topmost layer (5) being light-impermeable and having an annular opening of the diameters (D2 and D3) and a central opening of the diameter (D1), and below the topmost layer (5) a photosensitive layer (6) with a central concentric opening of a diameter between (D1 and D2), and therebelow a photosensitive layer (7), the diameters (D1 and D2, D3, respectively) being so selected that the layer (7) detects the central beam and the layer (6) substantially completely detects scattered radiation arriving in the scattering angle range underlying the measurement.

22. Apparatus according to one of claims 15 to 21, characterized by
- a servo amplifier (9) for the light source (10),
- one or more differential amplifiers (16) delivering from the measured signals of the light detectors (12)

17

signals corresponding to the relative intensity of the associated scattered light which are converted to corresponding digital signals in an A/D converter (20),

- A/D converters (22) converting the measured signals from the light detectors (12), one of which detects the intensity of the central beam and the others detect the intensity of the scattered radiation emanating from the sample (11), after amplification in signal amplifiers (13, 14, 15), to corresponding digital signals, and

- a measured value processing unit (17) which, on the basis of predetermined functions or programs, produces at the digital signals from the light detectors (12), or from the differential amplifier(s) (16), output signals for a display means (18) or for an output means (19) with D/A converter (23) before the input, and further produces an output quantity which, after conversion to an analog signal in a D/A converter (21), is delivered to the servo amplifier (9).

23. Apparatus according to one of claims 15 to 21, characterized by

- a servo amplifier (9) for the light source (10),

- a differential amplifier with integrator (22') integrating the difference signal of the measured signals (a, b) of two light detectors (12) one of which detects the intensity of the central beam and the other one the intensity of the scattered radiation emanating from the sample (11),

- two serially connected controllable amplifiers (23', 24) further amplifying the output signal from the differential amplifier with integrator (22'),

- a ratio former (25) forming the ratio (a/b) of the two measured signals (a, b) and controlling with a corresponding output signal the amplifier (23'),

- an amplifier (13) amplifying the measured signal (a),
and

- a differential amplifier (16) which compares the output signal from the amplifier (13) with a predetermined reference voltage, and whose output signal controls the amplifier (24).

24. Apparatus according to one of claims 15 to 23, characterized by a laser diode as light source.

25. Apparatus according to one of claims 15 to 24, characterized by phototransistors as light detectors for measuring the intensity of the central beam and of the scattered radiation.

26. Apparatus according to one of claims 15 to 25, characterized by a microcomputer or microprocessor as or in the signal processing unit, respectively.

27. Apparatus according to one of claims 15 to 26, characterized by a recorder, a plotter, or a miniature printer, optionally combined with a pocket calculator, as output means (19).

28. Apparatus according to one of claims 15 to 27, characterized by a means giving off an acoustical, optical and/or electrical signal when a predetermined upper and/or lower limit of the measured value is reached.

29. Apparatus according to one of claims 15 to 28, characterized in that it is microminiaturized.

30 Apparatus according to one of claims 15 to 29, characterized in that it is designed for controlling an infusion device.

31. Apparatus according to claim 30, characterized in that it is designed for controlling an insulin infusion device.

32. Apparatus according to one of claims 15 to 31, characterized by a temperature measuring sensor for measuring the skin temperature, and a means for correcüing the measured signal according to a predetermined correction function on the basis of the detected skin temperature.

**Revendications**

1. Procédé pour la détermination quantitative de la concentration de substances à bas ou haut poids moléculaire en solution, d'éléments figurés et de cellules du sang dans des systèmes à plusieurs composants, ces derniers différant suffisamment par les dimensions linéaires des molécules, leur structure et/ou composition et/ou la masse molaire, par:

a) diffusion de la lumière par l'échantillon et analyse de la forme de la courbe de diffusion avec détermination pour chaque composant à déterminer d'au moins une plage angulaire de diffusion sur laquelle la variation de l'intensité du rayonnement diffusé en fonction de la concentration du composant considéré est maximale;

b) correction de l'intensité mesurée du rayonnement diffusé considéré par l'intensité mesurée du rayon central;
et

c) affectation de la grandeur mesurée corrigée à la concentration du composant à déterminer.

2. Procédé selon revendication 1, caractérisé par la mesure de l'intensité du rayonnement diffusé avec variation continue de l'angle de diffusion.

3. Procédé selon revendication 1, caractérisé par mesure de l'intensité du rayonnement diffusé sous un angle de diffusion prédéterminé ou sur une plage angulaire de diffusion prédéterminée.

4. Procédé selon revendication 1, caractérisé par la mesure de l'intensité du rayonnement diffusé sous plusieurs angles de diffusion prédéterminés discrets ou sur plusieurs plages angulaires de diffusion, avec le cas échéant détermination simultanée de plusieurs composants d'une solution par un dépouillement approprié.

5. Procédé selon une quelconque des revendications 1 à 4, caractérisé par la correction du signal de mesure pour une épaisseur d'échantillon effective variable, telle que la correction d'irrigation aanguine, la grandeur de correction étant déduite de l'intensité mesurée du rayon central.

6. Procédé selon une quelconque des revendications 1 à 5, caractérisé en ce que la correction du signal de mesure est effectuée numériquement, de façon que sa nature soit fixée par une fonction mathématique et sa valeur prédéterminée par l'introduction de constantes.

7. Procédé selon une quelconque des revendications 1 à 5, caractérisé en ce que la correction du signal de mesure est effectuée analogiquement par une commande de gain appropriée et par comparaison à une valeur de consigne ajustable, telle que la tension aux bornes d'un potentiomètre.

8. Procédé selon une quelconque des revendications 1 à 7, caractérisé par l'emploi d'une source lumineuse à rayonnement polarisé linéairement, elliptiquement ou circulairement.

9. Procédé selon une quelconque des revendications 1 à 8, caractérisé en ce que le traitement des signaux est effectué analogiquement pendant les phases (b) et (c).

10. Procédé selon une quelconque des revendications 1 à 9, caractérisé en ce que la valeur mesurée du rayonnement diffusé, après la correction à l'aide de l'intensité du rayon central, est corrigée en fonction d'une ou plusieurs intensités relatives.

11. Procédé selon une quelconque des revendications 1 à 9, caractérisé par l'application d'une méthode à deux ou plusieurs signaux, puis correction de l'intensité mesurée du rayonnement diffusé par formation de la différence par rapport à une valeur de référence, en fonction d'une ou plusieurs intensités relatives et/ou un ou plusieurs produits d'intensités.

12. Procédé selon une quelconque des revendications 1 à 11, caractérisé par la prise de moyenne des valeurs mesurées avant ou après la correction.

13. Procédé selon une quelconque des revendications 1 à 12, caractérisé par l'emploi d'un seul photodétecteur pour la mesure de l'intensité du rayonnement diffusé et du rayon central, l'angle de la source lumineuse par rapport à l'échantillon étant modifié par déplacement de la source lumineuse et/ou par des miroirs déflecteurs mobiles.

14. Procédé selon une quelconque des revendications 1 à 13, caractérisé par l'insertion entre l'échantillon et le ou les photodétecteurs de filtres qui améliorent la sensibilité de mesure et réduisent le rayonnement parasite diffuse.

15. Dispositif pour la mise en oeuvre du procédé selon une quelconque des revendications 1 à 14, comprenant:
- une source lumineuse
- une partie échantillon
- un ou plusieurs photodétecteurs qui déterminent l'intensité du rayon central et du rayonnement diffusé sur une plage angulaire prédéterminée au moins
- un dispositif électronique de traitement des signaux pour correction des signaux de mesure
- une alimentation
et
- un dispositif d'indication ou de sortie du signal, et caractérisé par:
- un support (T) sensiblement en U avec à sa partie inférieure une articulation à ressort (G) qui repousse les deux parties latérales du support l'une vers l'autre en maintenant entre elles une distance permettant d'insérer et de maintenir un lobe d'oreille humaine entre les deux parties latérales du support
- une diode laser (D) conitituant la source lumineuse et disposée sur ou dans une partie latérale du support (T), le cas échéant dans un radiateur (K)
- un miroir déflecteur (U) disposé au-dessus de la diode laser (D) et déviant d'environ 90° la lumière émise par ladite diode, suivant une direction sensiblement horizontale et
- une partie de réception optique (E), prévue en regard du miroir déflecteur (U) sur l'autre partie latérale du support, avec au moins un photodétecteur qui détermine, sur une plage angulaire prédéterminée au moins, l'intensité du rayon central et du rayonnement diffusé de la lumière de la diode laser (D) transmise ou diffusée par le lobe inséré dans le support (T), et délivre des signaux électriques correspondants
- le dispositif électronique de traitement des signaux, le dispositif d'indication des signaux et/ou le dispositif de sortie des signaux ainsi que l'alimentation étant intégrés chacun au support ou indépendants de ce dernier.

16. Dispositif selon revendication 11, caractérisé par une partie de réception optique (E), réalisée de façon à déceler séparément deux plages angulaires par sectorisation.

17. Dispositif selon une des revendications 15 ou 16, caractérisé en ce que la partie de réception optique (E) est réalisée sous forme d'un conduit optique.

18. Dispositif selon une quelconque des revendications 15 à 17, caractérisé par une partie de réception optique (E) avec un boîtier (5p), dont la surface de base comprend un photodétecteur (2p) concentrique et dont la surface intérieure, vue du photodétecteur (2p), s'évase coniquement vers l'extérieur; un corps de verre annulaire (3p), disposé concentriquement à la surface conique, du côté ouvert du boîtier (5p), réalisé en verre ou un autre matériau transparent et dont la surface intérieure vue du photodétecteur (2p) diminue coniquement vers l'extérieur; une douille (4p) opaque au rayonnement ou munie d'un revêtement opaque au rayonnement, disposée concentriquement dans le corps de verre (3p) et dont l'extrémité extérieure est obturée là l'exception d'une ouverture centrale; et un photodétecteur (1p) disposé concentriquement dans la douille (4p) et suivant l'axe optique du montage, le diamètre (D1) de l'ouverture centrale de la douille (4p), le diamètre (D2) de la base extérieure de la douille (4p) et le diamètre (D3) de la base extérieure du corps de verre (3p) ainsi que le

**0 074 428**

demi-angle au sommet ($\alpha$) du cône de la douille (4p) et le demi-angle au sommet ($\beta$) du cône du boîtier (5p) ou du cône intérieur du corps de verre (3p) étant dimensionnés de façon que le photodétecteur (2p) détecte tout le rayonnement diffusé sur la plage angulaire de diffusion choisie.

19. Dispositif selon revendication 18, caractérisé en ce que la surface intérieure du boîtier (5p) ou la surface extérieure du corps de verre (3P) et/ou la surface extérieure de la douille (4p) ou la surface intérieure du corps de verre (3p) présentent une section à courbure parabolique.

20. Dispositif selon une des revendications 15 ou 16, caractérisé par une partie de réception optique plane, constituée par quatre couches (1 à 4) superposées, la couche inférieure (1) étant photosensible; la deuxième couche (2) située en dessus étant opaque au rayonnement et présentant une ouverture centrale de diamètre (D1) suivant l'axe optique; la troisième couche (3) située en dessus étant photosensible et présentant une ouverture concentrique centrale de diamètre (D2); et la quatrième couche (4) située en dessus présentant une ouverture centrale concentrique de diamètre (D3), les diamètres (D1 ou D2 et D3) étant choisis de façon à assurer une détection aussi totale que possible du rayon central par la couche (1) et du rayonnement diffusé sur la plage angulaire de diffusion adoptée pour la mesure par la couche (3).

21. Dispositif selon une des revendications 15 ou 16, caractérisé par une partie de réception optique plane, constituée par trois couches (5 à 7) superposées, la couche supérieure (5) étant opaque et présentant une ouverture annulaire de diamètres (D2 et D3) et une ouverture centrale de diamètre (D1); une couche photosensible (6) étant prévue sous la couche supérieure (5) avec une ouverture concentrique centrale de diamètre compris entre (D1 et D2); et une couche photosensible (7) étant prévue sous la couche (6), les diamètres (D1 ou D2 et D3) étant choisis de façon à assurer la détection aussi totale que possible du rayon central par la couche (7) et du rayonnement diffusé sur la plage angulaire de diffusion adoptée pour la mesure par la couche (6).

22. Dispositif selon une quelconque des revendications 15 à 21, caractérisé par:
- un amplificateur-régulateur (9) pour la source lumineuse (10)
- un ou plusieurs amplificateurs différentiels (16) qui, à partir des signaux de mesure des photodétecteurs (12), délivrent des signaux correspondant à l'intensité relative de la lumière diffusée affectée et qu'un convertisseur A/N (20) convertit en signeux numériques correspondants
- des convertisseurs A/N (22), qui convertissent les signaux de mesure des photodétecteurs (12), dont un décèle l'intensité du rayon central et les autres intensités du rayonnement diffusé par l'échantillon (11), en signaux numériques correspondants, après amplification dans les amplificateurs de signal (13, 14, 15), et
- une partie de traitement des valeurs de mesure (17) qui, à l'aide de fonctions prédéterminées ou de programmes et à partir des signaux numériques des photodétecteurs (12) ou du ou des amplificateurs différentiels (16), délivre des signaux de sortie à un dispositif indicateur (18) ou à un dispositif de sortie (19) avec convertisseur N/A (23) en amont, ainsi qu'une grandeur réglée qui, après conversion en un signal analogique par un convertisseur N/A (21) est appliquée à l'amplificateur-régulateur (9).

23. Dispositif selon une quelconque des revendications 15 à 21, caractérisé par:
- un amplificateur-régulateur (9) pour la source lumineuse (10)
- un amplificateur différentiel avec intégrateur (22'), qui intègre le signal différentiel des signaux de mesure (a, b) de deux photodétecteurs (12), dont un décèle l'intensité du rayon central et l'autre l'intensité du rayonnement diffusé par l'échantillon (11)
- deux amplificateurs commandés (23', 24) branchés en série et qui amplifient de nouveau le signal de sortie de l'amplificateur différentiel à intégrateur (22')
- un logomètre (25), qui forme le rapport (a/b) des deux signaux de mesure (a, b) et commande l'amplificateur (23') par un signal de sortie approprié,
- un amplificateur (13) qui amplifie le signal de mesure (a) et
- un amplficateur différentiel (16), qui compare le signal de sortie l'amplificateur (13) à une tension de référence prédéterminée, et dont le signal de sortie commande l'amplificateur (24).

24. Dispositif selon une quelconque des revendications 15 à 23, caractérisé par l'emploi d'une diode laser comme source lumineuse.

25. Dispositif selon une quelconque des revendications 15 à 24, caractérisé par l'emploi de phototransistors comme photodétecteurs pour la mesure de l'intensité du rayon central et du rayonnement diffusé.

26. Dispositif selon une quelconque des revendications 15 à 25, caractérisé par l'emploi d'un micro-ordinateur ou d'un microprocesseur comme partie de traitement de signaux ou dans cette dernière.

27. Dispositif selon une quelconque des revendications 15 à 26, caractérisé par l'emploi d'un enregistreur, d'un traceur ou d'une mini-imprimante, en liaison le cas échéant avec une calculatrice de poche, comme dispositif de sortie (19).

28. Dispositif selon une quelconque des revendications 15 à 27, caractérisé par un dispositif qui déclenche un signal acoustique, optique et/ou électrique quand un seuil supérieur et/ou inférieur prédéterminé de la valeur mesurée est atteint.

29. Dispositif selon une quelconque des revendications 15 à 28, caractérisé en ce qu'il est microminiaturisé.

30. Dispositif selon une quelconque des revendications 15 à 29, caractérisé par sa réalisation pour la commande d'un dispositif d'infusion.

31. Dispositif selon revendication 30, caractérisé par sa réalisation pour la commande d'un dispositif d'infusion d'insuline.

32. Dispositif selon une quelconque des revendications 15 à 31, caractérisé par un capteur pour la mesure de la température cutanée et un dispositif pour la correction du signal de mesure selon une fonction de correction prédéterminée, à partir de la température cutanée déterminée.

Fig. 1

Fig. 2

Fig. 3

Fig. 4 a

Fig. 4b

Fig. 4c

Fig. 4

7

Fig. 5

0074 428

Fig. 6

Fig. 7

Fig. 8

# 0 074 428

Fig. 9b

Fig. 9a

Fig. 9

17

Fig. 1c